# EUROPEAN PATENT SPECIFICATION

(11) **EP 3 897 688 B1**
(45) Date of publication and mention of the grant of the patent: **07.06.2023**
(21) Application number: 19832342.0
(22) Date of filing: 18.12.2019
(51) Int. Cl.: A61K 38/17, A61P 31/10

(54) **USE OF SAP FOR THE TREATMENT OF EUROTIOMYCETES FUNGI INFECTIONS**
VERWENDUNG VON SAP ZUR BEHANDLUNG VON EUROTIOMYCETES-PILZINFEKTIONEN
UTILISATION DE SAP POUR LE TRAITEMENT D'INFECTIONS PAR DES CHAMPIGNONS EUROTIOMYCETES

(30) Priority: 20.12.2018 EP 18214853
(43) Date of publication of application: 27.10.2021
(73) Proprietor: Humanitas Mirasole S.p.A., 20089 Rozzano (MI) (IT); Humanitas University, 20090 Pieve Emanuele (MI) (IT)
(72) Inventor: DONI, Andrea, 20089 ROZZANO (MI) (IT); MANTOVANI, Alberto, 20090 PIEVE EMANUELE (MI) (IT)
(74) Representative: Capasso, Olga
(86) International application number: PCT/EP2019/085932
(87) International publication number: WO 2020/127471

(56) References cited:
- WO-A1-2005/107791
- WO-A2-2009/009034
- US-A1- 2010 323 970
- MOREIRA A P ET AL: "Serum amyloid P attenuates M2 macrophage activation and protects against fungal spore-induced allergic airway disease", JOURNAL OF ALLERGY AND CLINICAL IMMUNOLOGY, ELSEVIER, AMSTERDAM, NL, vol. 126, no. 4, 1 October 2010 (2010-10-01), pages 712-721.e7, XP027422131, ISSN: 0091-6749, DOI: 10.1016/J.JACI.2010.06.010 [retrieved on 2010-07-31]
- GARLANDA C ET AL: "Non-redundant role of the long pentraxin PTX3 in anti-fungal innate immune response", NATURE, MACMILLAN JOURNALS LTD, LONDON, vol. 420, no. 6912, 14 November 2002 (2002-11-14), pages 182-186, XP002337598, ISSN: 0028-0836, DOI: 10.1038/NATURE01195
- GARCIA-SHERMAN MELISSA C ET AL: "A unique biofilm in human deep mycoses: fungal amyloid is bound by host serum amyloid P component", NPJ BIOFILMS AND MICROBIOMES, NATURE PUBLISHING GROUP (NPG) , vol. 1, no. 1 1 January 2015 (2015-01-01), pages 1-4, XP009513994, ISSN: 2055-5008, DOI: 10.1038/NPJBIOFILMS.2015.9 Retrieved from the Internet: URL:https://www.ncbi.nlm.nih.gov/pmc/artic les/doi/10.1038/npjbiofilms.2015.9

## Description

This invention relates to the use of Serum Amyloid P component (SAP) polypeptides for the treatment of *Eurotiomycetes* fungi infections, in particular for aspergillosis and invasive aspergillosis, alone or in combination with pentraxin-3 (PTX3) polypeptides.

### BACKGROUND OF THE INVENTION

Aspergillus fungi are representatives of the *Trichocomaceae* family of the *Eurotiales* order, which in turn belong to the *Eurotiomycetes* class.

Aspergillosis is an opportunistic fungus infection, most often the consequence of an *Aspegillus Fumigatus* infection, associated with a wide spectrum of diseases in humans, ranging from severe infections to allergy in immune-compromised patients (Lionakis *et al.,* 2018). In particular, aspergillosis is a major life-threatening infection patients with impaired phagocytosis, for instance, during chemotherapy or radiotherapy-induced neutropenia (Cunha *et al.,* 2014), because their reduced immunity allows for the infection to spread from the lungs to other major organs, leading to a condition called invasive aspergillosis.

The innate immune system represents the first line of resistance against pathogens and a key determinant in the activation and orientation of adaptive immunity through the complementary activities of a cellular and humoral arm (Bottazzi *et al.,* 2010). Cell-associated innate immune molecules sense pathogen-derived agonists leading to activation of different inflammatory pathways (Inohara *et al.,* 2005; Takeda *et al.,* 2003), which include phagosome formation (Sanjuan *et al.* ,2009). Humoral & Pattern Recognition Molecules (PRMs) are an essential components of the innate immune response sharing functional outputs with antibodies (Bottazzi *et al.,* 2010; Mantovani *et al.,* 2013) including opsonisation, regulation of complement activation, agglutination and neutralization, discrimination of self versus non-self and modified-self (Bottazzi *et al.,* 2010). Humoral PRMs in turn interact with and regulate cellular effectors (Bottazzi *et al.,* 2010; Lu *et al.,* 2008; Lu *et al.*,2012; Hajishengallis *et al.,* 2010) collaborating to form stable pathogen recognition complexes for pathogen clearance (Bottazzi *et al.,* 2010; Ng *et al.,* 2007; Ma *et al.,* 2009; Ma *et al.,* 2011). These include complement cascade molecules (Ricklin *et al.,* 2013 ; Genster *et al.,*2014)*,* ficolins (Fujita *et al.,* 2002), collectins (Holmskov *et al.,* 2003) and pentraxins (Lu *et al.,*2012; Bottazzi *et al.,* 2016; Pepys *et al.,* 2003).

Pentraxins consists of an ancient group of proteins evolutionarily conserved from arachnids and insects to humans characterized by the presence of a 200 amino acid (aa) pentraxin domain in their carboxyl-terminal and a pentraxin signature (HxCxS/TWxS, x=any aa) (Pepys *et al.,* 2003; Garlanda *et al.,* 2005; Mantovani *et al.,* 2008; Szalai *et al.,* 1999; Du Clos *et al.,*2011). Human C Reactive protein (CRP, also called PTX1) and SAP (PTX2) constitute the short pentraxin arm of the superfamily. Human CRP and SAP share gene localization and organization, protein structure and protein sequence identity (51% of aa identity). Human and murine SAP diverge in protein sequence (66% of aa identity) and regulation (Lu *et al.,* 2008; Emsley *et al.,*1994). CRP and SAP are acute phase response proteins produced in the liver in response to infections and inflammatory cytokines, respectively in human and mouse (Casas *et al.,* 2008; Pepys *et* al,1979). Extra hepatic sources of short pentraxins have been described but without contributing to blood levels (Pepys *et al.,* 2003).

PTX3 differs from the classical short pentraxins on the basis of gene localization and regulation, protein structure, and cellular sources (Bottazzi *et al.,* 2016). PTX3 is highly conserved in human and mouse (92% of aa residue identity) and is similarly induced in immune cells (e.g. dendritic cells, macrophages) and stromal cells in response to local proinflammatory signals and pathogens (Bottazzi *et al.,* 2016; Garlanda *et al.,* 2005). PTX3 is stored in neutrophil granules and promptly released upon their activation (Jaillon *et al.,* 2007). Studies in gene-targeted mice and in humans proved an essential role of PTX3 in innate immune responses against certain pathogens (Garlanda *et al.,* 2002; Jaillon *et al.,* 2014; Jeannin *et al.,* 2005; Wojtowicz *et al.,* 2015; Olesen *et al.,* 2007; Magrini *et al.,*2016). In particular, mechanisms underlying the PTX3-mediated resistance to *A. fumigatus* were extensively investigated (Garlanda *et al.,* 2002; Moalli *et al.,* 2010). An association between genetic variants of PTX3 and occurrence of invasive aspergillosis after allogeneic hematopoietic stem-cell transplantation in humans is consolidated (Cunha *et al.,* 2014; Cunha *et al.,* 2015; Fisher *et al.,* 2017; Lionakis *et al.,* 2018).

CRP was the first pentraxin identified as a prototypic PRM in the 1940 and subsequently described to bind various microorganisms including fungi, yeasts, bacteria and parasites (Szalai *et al.,* 2002). *In vitro* studies also indicate a specific interaction of SAP with a wide range of microorganisms, including Gram-positive (An *et al.,* 2013 ; Yuste *et al.,* 2007) and Gram-negative (Noursadeghi *et al.,* 2000) bacteria and influenza virus (Andersen *et al.,* 1997), through recognition of moieties such as phosphorylcholine (PC) (Schwalbe *et al.,* 1992), teichoic acid (An *et al.,* 2013) and terminal mannose or galactose glycan residues (Hind at al., 1985). CRP and SAP also interact with complement components to boost innate response to pathogens (Du Clos et al., 2011; Ma *et al.,* 2017; Doni *et al.,* 2012). However, because of considerable divergence in regulation between mouse and man (Pepys *et al.,* 2003), studies on the physiological relevance of CRP and SAP are not conclusive. Indeed, SAP is constitutively found in human blood, but it does not increase upon inflammatory stimuli (Szalai *et al.,*1999), whereas it is the main acute-phase reactant in mice (Pepys et al., 1979). CRP is instead a major acute phase protein only in humans (Pepys *et al.,* 2003). Thus, observations related to functions of the short pentraxins in mice are more difficult to be extrapolated (Pepys *et al.,* 2006; Tennent *et al.,* 2008).

### PRIOR ART

The recombinant of endogenous human SAP (PRM-151) has been proposed as a novel anti-fibrotic immunomodulator in patients with Idiopathic Pulmonary Fibrosis (IPF) in placebo-controlled Phase 2 study trial (van den Blink *et al.,* 2016)

A discrepancy between *in vitro* and *in vivo* results exists on the role of SAP in innate immunity. SAP prevented *in vitro* cell infection by influenza A virus (Andersen *et al.,* 1997), and intracellular growth of mycobacteria (Singh *et al.,* 2006) and malaria parasites (Balmer et al., 2000), thus suggesting a protective role in influenza, tuberculosis and malaria. However, *in vivo* relevance of SAP in influenza A infection is controversial (Herbert et al., 2002 ; Job *et al.,* 2013), nor SAP effect on pulmonary innate immunity against tuberculosis or malaria is reported. SAP acted as opsonin for *Streptococcus pneumonia* and improved complement deposition on bacteria thus promoting phagocytosis (Yuste *et al.,* 2007). SAP also enhanced *in vitro* phagocytosis of *zymosan* (Mold *et al.,* 2001; Bharadwaj *et al.,* 2001) and *Staphylococcus aureus* (An *et al.,* 2013) by neutrophils and macrophages through FcyR-dependent but complement-independent mechanisms. On the other hand, SAP was not opsonic for *Listeria monocytogenes* though it enhanced macrophage listericidal activity (Singh *et al.,* 1986). SAP interaction with certain microbes even resulted in anti-opsonic activity or in aiding virulence of these pathogens. SAP inhibited immune recognition of *Mycobacterium tuberculosis* by macrophages (Kaur *et al.,* 2004). Interaction of SAP with *S. pyogenes, Neisseria meningitides* and some variants of *Escherichia coli* led to decreased phagocytosis and killing by macrophages and inhibition of complement activation (Noursadeghi *et al.,* 2000), and SAP-deficient mice showed higher survival in experimental infections with *S. pyogenes* and *E. coli* (Noursadeghi *et al.,* 2000). SAP was found in autopsy tissues of patients affected by invasive gastrointestinal candidiasis associated with fungus (Gilchrist *et al.,* 2012). Classical and lectin pathways are both main initiators of complement activation against *A. fumigatus* (Rosbjerg *et al.,* 2016). Heterocomplex of mannose-binding lectin (MBL) and SAP triggers cross-activation of complement on *Candida albicans* (Ma *et al.,* 2011). SAP binds to lipopolysaccharide (LPS) but does not regulate inflammation in experimental endotoxemia (Noursadeghi *et al.,* 2000; de Haas *et al.,* 2000). Recent indirect evidence suggest an interaction of SAP with filamentous forms of invading fungi (Garcia-Sherman *et al.,* 2015). SAP was indeed found in autopsy tissues of patients affected by invasive gastrointestinal candidiasis (Gilchrist *et al.,* 2012) and aspergillosis, mucormycosis, and coccidioidomycosis (Klotz *et al.,* 2016). Moreover, SAP administration inhibited the FcyR-mediated alternative macrophage activation dampening the allergic airway disease induced by *A. fumigatus,* in which an airway hyper reactivity and a THz cytokine profile contribute to alternative activation of macrophages that exhibit impaired clearance of fungi (Moreira *et al.,* 2010). SAP was also suggested as ligand for DC-SIGN (CD209; mouse SIGN-R1) on neutrophils and macrophages in the context of fibrosis (Cox *et al.,* 2015).

To our knowledge, no relevance of SAP in antifungal innate immune response is reported.

### BRIEF DESCRIPTION OF THE DRAWINGS

Fig. 1 depicts the results of intratracheal (i.t.) injection of *A. fumigatus* conidia in mice
Fig. 2 depicts the susceptibility of SAP-deficient mice to *A. fumigatus*
Fig. 3 depicts the inflammatory response to *A. fumigatus*
Fig. 4 depicts inflammatory response in injured tissue
Fig. 5 depicts the rescue of susceptibility to *A. fumigatus* in SAP-deficient mice
Fig. 6 depicts the effect of SAP on complement activation on *A. fumigatus*
Fig. 7 depicts the effect of SAP on *A. fumigatus* phagocytosis by neutrophils and the effect of human SAP on phagocytic activity
Fig. 8 depicts the initiation of classical complement activation at the bases of SAP-mediated phagocytosis
Fig. 9 depicts how SAP effect on phagocytosis is independent from its interaction with DC-SIGN
Fig. 10 depicts the susceptibility to *A. fumigatus* associated with single or double deficiency for SAP and PTX3
Fig. 11 depicts levels of SAP in patients affected by invasive aspergillosis
Fig. 12 depicts the therapeutic efficacy of SAP in treatment of invasive aspergillosis
Fig. 13 depicts the therapeutic efficacy of SAP in the treatment of invasive aspergillosis alone or in combination with posaconazole
Fig. 14 depicts the determination of SAP-mediated killing of *A. fumigatus* conidia.
Fig. 15 depicts the susceptibility of SAP-deficient mice to *A. flavus*
Fig. 16 depicts the binding of Sap on Candida and the susceptibility of SAP-deficient mice to Candida *albicans* bloodstream infection

### DETAILED DESCRIPTION OF THE INVENTION

We have surprisingly found that SAP is involved in the innate immune response against Aspergillus fungi and that classical complement activation is required for the initiation of SAP-mediated phagocytosis of these fungi. By interacting with Aspergillus, SAP triggers complement-mediated inflammatory and innate responses essential for pathogen clearance. Use of SAP can trigger a complement-mediated fluid-phase innate immune response aimed at a microbicidal effect inducing assembly of the terminal membrane lytic complex on fungal surface via the classical complement activation pathway, and hence the basis of a novel therapeutic use of SAP, particularly in therapy-induced immunocompromised patients.

Accordingly, under a first aspect of this invention there is provided a SAP polypeptide or a functional fragment of such SAP polypeptide for use in the treatment of a *Eurotiomycetes* fungus infection.

In one embodiment, the *Eurotiomycetes* fungus is an *Eurotiales* fungus.

In a particular embodiment, the *Eurotiales* fungus is a *Trichocomaceae* fungus.

In a more particular embodiment, the *Trichocomaceae* fungus is infection is aspergillosis.

As used herein, the term "aspergillosis" excludes the merely inflammatory manifestations of aspergillosis like allergic bronchopulmonary aspergillosis and severe asthma sensitized to Aspergillus and includes all life-threatening generalised infections caused by Aspergillus in subjects with compromised immune systems: aspergilloma and chronic pulmonary aspergillosis in subjects previously affected by tuberculosis or sarcoidosis; aspergillus bronchitis in subjected affected by bronchiectasis or by cystic fibrosis; aspergillus sinusitis; and all of these diseases that evolve to invasive aspergillosis in subjects with low immune defenses such as in bone marrow transplant, chemotherapy for cancer treatment, AIDS, major burns, and in chronic granulomatous disease.

In a particular embodiment, the aspergillosis is an invasive aspergillosis.

In a further embodiment, the aspergillosis or invasive aspergillosis is due to an *Aspergillus Fumigatus* infection.

In a further embodiment, the aspergillosis or invasive aspergillosis is due to an *Aspergillus Flavus* infection.

As used herein "The percent (%) amino acid sequence identity" with respect to a reference polypeptide sequence is defined as the percentage of amino acid residues in a candidate sequence that are identical to the amino acid residues in the reference polypeptide sequence, after aligning the sequences and introducing gaps, if necessary, to achieve maximum percent sequence identity, and not considering any conservative substitutions as part of the sequence identity. The alignment in order to determine the percent of amino acid sequence identity can be achieved in various ways that are within the skill in the art, for instance, using publicly available computer software such as BLAST, BLAST-2, ALIGN or Megalign software (DNASTAR). Those skilled in the art can determine appropriate parameters for aligning sequences, including any algorithms needed to achieve maximal alignment over the full-length of the sequences being compared.

As used herein, a "functional fragment" of a SAP polypeptide is a portion of the SAP polypeptide that retains at least 70% native SAP activity in an assay suitable to test for its pharmacological activity , in particular a test useful for determining its activity in the treatment of a *Eurotiomycetes* fungus infection. In one embodiment, the SAP polypeptide functional fragment retains at least a percentage of native SAP activity selected from the list of 75%, 80%, 85%, 90% and 95%.

As used herein the term "SAP polypeptide" encompasses all functional forms, derivatives and variants of SEQ ID NO: 1, i.e. not limitedly:
- glycosylated forms such as that can be purified from human serum, which bears an N-linked oligosaccharide chain, wherein at least one branch of the oligosaccharide chain terminates with a α 2,3-linked sialic acid moiety, but also other functional glycosylated forms
- any recombinant human SAP, such as the recombinant human SAP known as PRM-151 (Duffield and Lupher, 2010)
- derivatives of SEQ ID NO: 1 that comprise modified amino acid residues such as PEGylated, prenylated, acetylated, biotinylated amino acids and the like.
- naturally found variants of SEQ ID NO: 1 such as the ones described and referred to in Kiernan *et al.,* 2004.

In another embodiment, the SAP polypeptide comprises an amino acid sequence that is at least identical to SEQ ID NO: 1 in a percentage selected from the list of 70%, 75%, 80%, 81%, 82%, 83%, 84%, 85%, 86%, 87%, 88%, 89%,90%, 91%, 92%, 93%, 94%, 95%, 96%, 97%, 98%, and 99%

In another embodiment, the SAP polypeptide comprises an amino acid sequence that is identical to SEQ ID NO: 1.

We have also determined that SAP and PTX3 share ability to interact with similar microorganisms and to act as opsonins via FcyR and that deficiency of both SAP and PTX3 entails a further increase of susceptibility to Aspergillus infection compared to that observed in mice with single deficiency. Therefore, our results indicate an additive role of SAP and PTX3 in the antifungal response at crossroad between complement and FcyR-mediated recognition (Lu *et al.,* 2008; Moalli *et al.,* 2010).

Accordingly, in a second aspect of this invention, there is provided the combination of a SAP polypeptide or a functional fragment of such SAP polypeptide with a PTX3 polypeptide or a functional fragment of such PTX3 polypeptide for use in the treatment of a *Eurotiomycetes* fungus infection.

In one embodiment, the *Eurotiomycetes* fungus is an *Eurotiales* fungus.

In a particular embodiment, the *Eurotiales* fungus is a *Trichocomaceae* fungus.

In a more particular embodiment, the *Trichocomaceae* fungus is infection is aspergillosis.

In a particular embodiment, the aspergillosis is an invasive aspergillosis.

In a further embodiment, the aspergillosis or invasive aspergillosis is due to an Aspergillus Fumigatus infection.

In a further embodiment, the aspergillosis or invasive aspergillosis is due to an Aspergillus Flavus infection.

As used herein, a "functional fragment" of a PTX3 polypeptide is a portion of the PTX3 polypeptide that retains at least 70% native PTX3 activity, in an assay suitable to test for its pharmacological activity in combination with a SAP polypeptide or functional fragment of such SAP polypeptide, in particular a test useful for determining its activity in the treatment of a *Eurotiomycetes* fungus infection when used in combination with a SAP polypeptide or a functional fragment of such SAP polypeptide. In one embodiment, the PTX3 polypeptide functional fragment retains at least a percentage of native PTX3 activity selected from the list of 75%, 80%, 85%, 90% and 95%.

As used herein the term "PTX3 polypeptide" encompasses all functional forms, derivatives and variants of SEQ ID NO: 2, i.e. not limitedly:
- any recombinant forms of PTX3 purified from supernatant of different cell sources, including Chinese hamster ovary (CHO) cell lines (Bottazzi *et al.,* 1997; Rivieccio *et al.,* 2007) and PerC6 (Marschner *et al.,* 2018);
- any isolated oligomeric forms of PTX3 (e.g. octameric, monomeric) (Cuello *et al.,* 2014);
- any recombinant glycosylated forms of PTX3 which bears N-linked fucosylated and sialylated complex-type sugars (Inforzato *et al.,* 2006), but also other functional glycosylated forms;
- any recombinant forms of PTX3 derived from non-synonymous single nucleotide polymorphisms (SNPs) in PTX3 gene (Thakur *et al.,* 2016), with the exception of the one derived from the SNP rs3816527 (Cunha *et al.,* 2014);
- derivatives of SEQ ID NO: 2 that comprise modified amino acid residues such as PEGylated, prenylated, acetylated, biotinylated amino acids and the like.

In another embodiment, the SAP polypeptide comprises an amino acid sequence that is at least identical to SEQ ID NO: 1 in a percentage selected from the list of 70%, 75%, 80%, 81%, 82%, 83%, 84%, 85%, 86%, 87%, 88%, 89%,90%, 91%, 92%, 93%, 94%, 95%, 96%, 97%, 98%, and 99%.

In another embodiment, the SAP polypeptide comprises an amino acid sequence that is identical to SEQ ID NO: 1.

In another embodiment, the PTX3 polypeptide comprises an amino acid sequence that is at least identical to SEQ ID NO:2 in a percentage selected from the list of 70%, 75%, 80%, 81%, 82%, 83%, 84%, 85%, 86%, 87%, 88%, 89%,90%, 91%, 92%, 93%, 94%, 95%, 96%, 97%, 98%, and 99%.

In another embodiment, the PTX3 polypeptide comprises an amino acid sequence that is identical to SEQ ID NO:2.

All embodiments may be combined.

### Formulation

The polypeptides of the invention may be administered in the form of tablets or lozenges formulated in a conventional manner. For example, tablets and capsules for oral administration may contain conventional excipients including, but not limited to, binding agents, fillers, lubricants, disintegrants and wetting agents. Tablets may be coated according to methods well known in the art.

The polypeptides of the invention may also be administered as liquid formulations including, but not limited to, aqueous or oily suspensions, solutions, emulsions, syrups, and elixirs. The y may also be formulated as a dry product for constitution with water or other suitable vehicle before use. Such liquid preparations may contain additives including, but not limited to, suspending agents, emulsifying agents, nonaqueous vehicles and preservatives.

The polypeptides of the invention may also be formulated as suppositories, which may contain suppository bases including, but not limited to, cocoa butter or glycerides.

The polypeptides of the invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane.

The polypeptides of the invention may also be formulated as transdermal formulations comprising aqueous or nonaqueous vehicles including, but not limited to, creams, ointments, lotions, pastes, medicated plaster, patch, or membrane.

The polypeptides of the invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents.

### Administration

Administration of the compositions using the method described herein may be orally, parenterally, sublingually, transdermally, rectally, transmucosally, topically, via inhalation, via buccal administration, or combinations thereof. Parenteral administration includes, but is not limited to, intravenous, intraarterial, intraperitoneal, subcutaneous, intramuscular, intrathecal, and intraarticular.

### Dosage

The therapeutically effective amount required for use in therapy varies with the nature of the condition being treated, and the age/condition of the patient. The desired dose may be conveniently administered in a single dose, or as multiple doses administered at appropriate intervals, for example as two, three, four or more sub-doses per day. Multiple doses may be desired, or required.

### EXAMPLES

The invention is now described by means of non-limiting examples.

### Example 1:

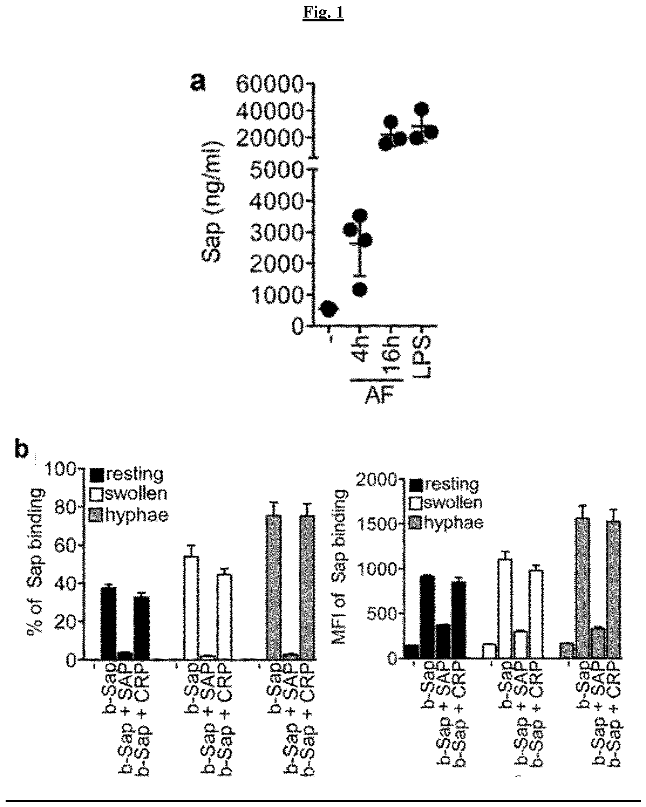

Fig. 1 depicts the interaction of SAP with A. *fumigatus* conidia in mice. a) induction of SAP circulating levels after i.t. injection of 5×10⁷ *A. fumigatus* (AF) conidia; LPS, 0.8mg/Kg. Mean±SD. c) FACS analysis of binding of biotin-conjugated (b-) murine SAP (Sap; 10µg/ml) to viable dormant or germinating conidia of AF (1×10⁸). Human SAP (50µg/ml) and CRP (50µg/ml) were also used. Mean±SD of one quadruplicate experiment of two performed.

Fig. 1a shows increased circulating levels of SAP (0.55±0.43 µg/ml; n=3) at 4 (2.63±1.02 µg/ml, n=4) and 16h (22.12±8.50 µg/ml, n=3) comparably to those observed after LPS administration (28.47±11.43 µg/ml, n=3; LPS, 0.8mg/Kg, 16h) Confocal microscopy analysis not shown here shows that in lungs (n=3; 4h), SAP localized areas of cell recruitment and complement deposition closely associated with conidia . This data also shows that recombinant murine SAP (Sap; 10 µg/ml) bound viable dormant, swollen and germinated conidia, as assessed by FACS (Fig. 1b) and confocal microscopy (data not shown here). Binding was competed by human SAP (50 µg/ml), but not CRP (50 µg/ml) (Fig. 1b), thus indicating that binding site on *A. fumigatus* is conserved between mouse and human SAP.

### Example 2:

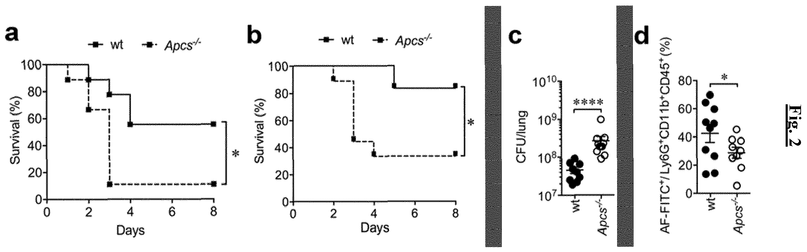

Fig. 2 depicts the susceptibility of SAP-deficient mice to *A. fumigatus.* a,b) survival of wt *and Apcs*^{*-*/*-*} mice after i.t. injection of 1×10⁸ (a) or 5×10⁷ (b) conidia; wt, n=9 (a) or n=6 (b); *Apcs*^{*-*/}*⁻,* n=9 (a, b). *, P<0.05 (Mantel-Cox test) (a, b). c) number of CFU per lung at 16h. Each spot corresponds to a single mouse. One experiment out of two performed with similar results. Mean±SEM. P<0.0001 (Mann-Whitney test). d, FACS analysis of *in vivo* phagocytosis in BALF neutrophils 4h after injection of 5×10⁷ fluorescein-labelled AF conidia. The figure shows results of two pooled experiments. Mean±SEM. *, P<0.05 (unpaired t-test).

Apcs^{-/-} mice showed lethal infection with a median survival time (MST) of 3 days compared to MST>10 of wt, both when 1x108 (Fig. 2a) or 5x107 (Fig. 2b) conidia were used. Actually, 89.9% (8/9) (Fig. 2a) and 44.4% (5/9) (Fig. 2b) of Apcs-/- mice succumbed on day 3 compared to 23.8% (2/9) and 0% (0/6) of wt mice. At the end of the experiment, 11.1% (1/9) and 33.3% (3/9) of Apcs^{-/-} mice survived to infection compared to 55.6% (5/9) and 83.3% (5/6) of wt mice, respectively when 1×10⁸ (Fig. 2a) or 5×10⁷ (Fig. 2b) conidia were used. In lung, susceptibility of Apcs-/- mice was associated with 6-fold increase of A. fumigatus CFU [median, 1.9x108, interquartile range (IQR) 3.0x108-1.6x108 vs. 4.0±x107, IQR 6.8x107-1.7x107; P<0.0001] (Fig. 2c) and reduced phagocytosis by neutrophils (28.74±3.96% vs. 42.53±6.39%; P=0.046) (Fig. 2d), considered as major players in the innate resistance against this fungus 57.

### Example 3:

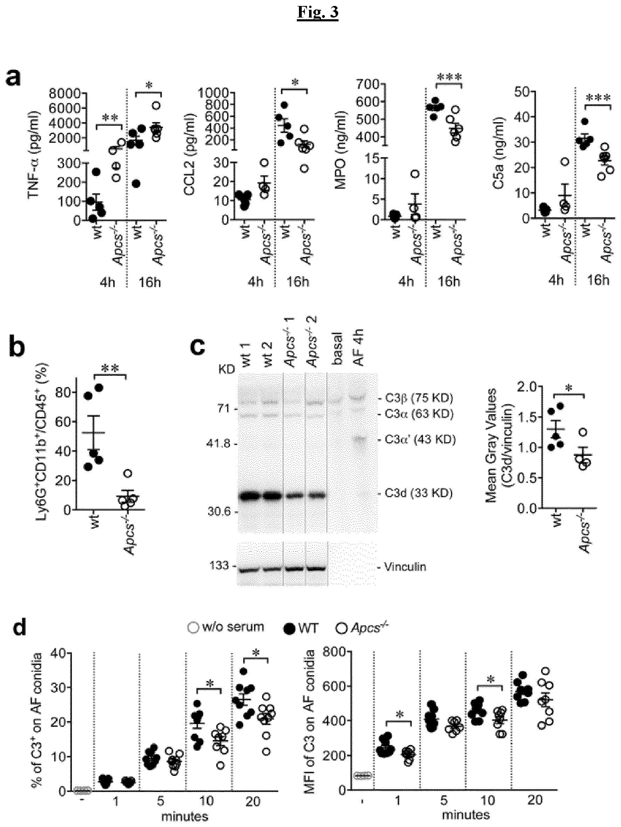

Fig. 3 depicts the inflammatory response to *A. fumigatus.* a) cytokines, Myeloperoxidase (MPO), C5a levels in BALFs after injection of 5×10⁷ AF conidia. One experiment out of two performed. Mean±SEM. *, P<0.05; **, P<0.01; ***, P<0.005 (Mann-Whitney test).b), FACS analysis of neutrophil recruitment in lung at 16h. One experiment shown out of two performed. Mean±SEM. **, P<0.01 (Mann-Whitney test). c) left, Western blot analysis of complement C3 fragments in lung lysates 4h after injection of 5×10⁷ AF conidia. N=5 wt and *n*=4 *Apcs*^{*-*/*-*} mice, two representative loading per genotype are shown (10µg/lane of proteins); 1µl/lane of mouse plasma in basal conditions and 4h after AF injection. Vinculin used as loading control is also shown. Right, results are expressed as mean±SEM grey values of C3d/vinculin. *, P<0.05 (unpaired t-test). d) FACS analysis of serum C3 deposition on AF conidia (1x10'). *, P<0.05 (unpaired t-test). One experiment out of four performed using serum or plasma.

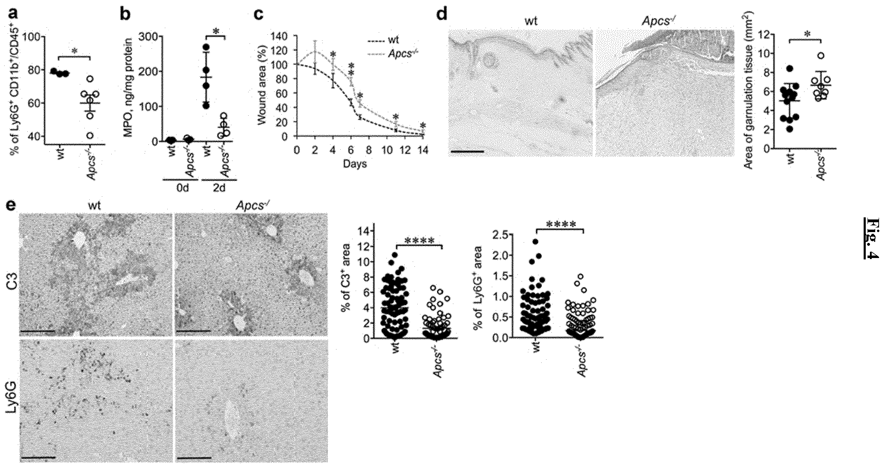

Fig. 4 depicts inflammatory response in injured tissue. a) FACS analysis of neutrophils recruitment at skin wound site (day 2). b) MPO content in wound lysates in normal skin or 2 days after injury, a, b, mean±SD; *, P<0.05 (Mann-Whitney test). c) kinetic analysis of skin excisional wound areas was performed. Values represent mean±SD. *, P<0.05; **, P<0.01 (unpaired t-test). One representative experiment (n=5 mice/group) out of 2 is shown. d) histological analyses of wound healing are shown at 14 days after wounding. Left, representative histological images (H&E; n=3-5 10X images per mouse). N=12 wt; n= 8 *Apcs*^{*-*/*-*} mice. Scale bar, 100µm. Right, measurement of wound granulation tissue by image analysis. Mean±SD *, P<0.05 (unpaired t-test). e) representative immunohistochemistry of C3 and Ly6G (left) in chemical-induced liver injury (8h) in wt (n=8) and *Apcs*^{*-*/*-*} (n=7) mice and quantification of the immunoreactive areas (right). Left, scale bar, 100µm. Right, 5-8 20X images per mouse; ****, P<0.0001 (unpaired t-test).

SAP regulates innate immune cell activities (Cox *et al.,* 2014; Cox *et al.,* 2015), thus affecting inflammatory reactions. In an effort to investigate whether a different inflammatory response was the bases of the phenotype associated to SAP-deficiency, cytokines were measured in the bronchoalveolar fluids (BALFs) in infected mice (Fig. 3a). At 4h, TNF-α increased in BALFs of *Apcs*^{*-*/*-*} mice compared to wt (552±278 vs. 95±43pg/ml; P=0.05). Levels of CCL2, myeloperoxidase (MPO) and C5a were low in both genotypes, however slightly increased in *Apcs*^{*-*/*-*} mice. At 16h, TNF-α levels remained higher *in Apcs*^{*-*/*-*} mice (3410±619 vs. 1672±555pg/ml; P=0.03), whereas CCL2 (127±55 vs. 444+114pg/ml; P= 0.01), MPO (449±28 vs. 564+16pg/ml; P=0.004) and C5a (22.7±1.7 vs. 31.5±1.8pg/ml; P= 0.003) were significantly lower compared to wt (Fig. 3a). At 16h, number of recruited neutrophils was also decreased in the lung of *Apcs*^{*-*/*-*} mice (P=0.008) (Fig. 3b). At 4h, a decreased C3d formation was observed in the lung lysates of *Apcs*^{*-*/*-*} mice (P=0.03) (Fig. 3c), therefore suggesting a dependence on impaired complement activation in the defective inflammatory response associated with SAP-deficiency. In agreement, a decreased deposition of C3 was *in vitro* observed on *A. fumigatus* conidia in the presence of serum of *Apcs*^{*-*/*-*} mice compared to wt (Fig. 3d). An impaired neutrophil recruitment and C3 deposition was also observed in wounds of *Apcs*^{*-*/*-*} mice in non-infectious models of skin (Fig. 4 a-d) and liver (Fig. 4e injury, defects possibly at the base of their delayed healing (Fig. 4c-d) (Martin *et al.,* 2005).

### Example 4:

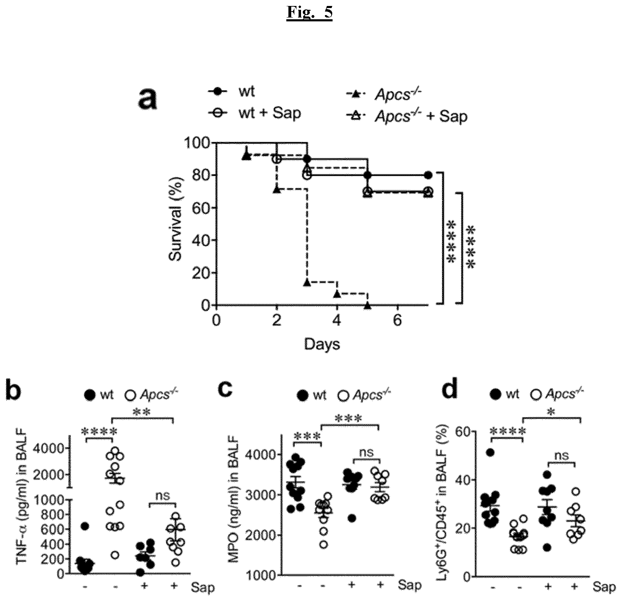

Fig. 5 depicts the rescue of susceptibility to *A. fumigatus* in SAP-deficient mice. a) b) c) d) i.t. injection of non-opsonised or Sap-opsonised AF conidia. a) survival of wt and *Apcs*^{*-*/*-*} mice. Wt, n=10; wt + Sap, n=10; *Apcs*^{*-*/}*⁻,* n=14; *Apcs*^{*-*/*-*} + Sap, n=13. ****, P<0.0001, wt vs. *Apcs*^{*-*/*-*} or *Apcs*^{*-*/*-*} vs. *Apcs*^{*-*/*-*} + Sap (Mantel-Cox test). b) and c) levels of TNF-α (b) and MPO (c) in BALFs of mice (16h). b), ****, P<0.0001; **, P<0.01. c), ***, P<0.0005. d, FACS analysis of neutrophil recruitment in lung (16h). Mean±SEM. ****, P<0.0001; *, P<0.05. b), c), d), Mean±SEM; Mann-Whitney test.

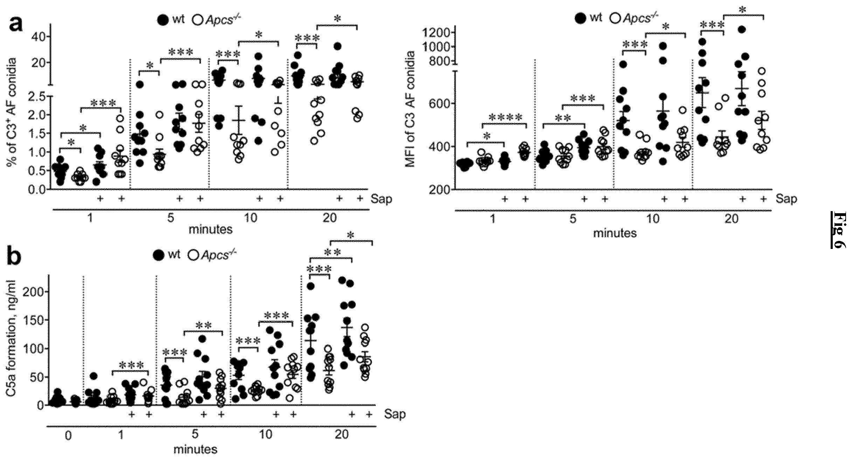

Fig. 6 depicts the effect of SAP on complement activation on *A. fumigatus.* a) FACS analysis of plasma C3 deposition on AF conidia (1×10⁷) opsonized or not with Sap. b) plasma C5a levels after incubation with AF conidia shown in a. a) and b) n=10 mouse plasma/genotype. b) representative experiment out of three performed. a) and b) Mean±SEM. *, P<0.05; **, P<0.01; ***, P<0.005; ****, P<0.0001 (unpaired t-test).

The pre-opsonisation of *A. fumigatus* conidia with recombinant murine SAP (1.10⁹/50µg; 5×10⁷ i.t. injected per mouse) rescued the susceptibility of *Apcs*^{*-*/*-*} mice to infection, without affecting the resistance of wt mice (Fig. 5a. Indeed, 69% (9/13) of SAP-treated *Apcs*^{*-*/*-*} mice resisted to infection (MST>10 days; P<0.0001) compared to 0% (0/14) of *Apcs*^{*-*/*-*} mice treated with vehicle (MST 3 days). Survival was similar in wt groups (wt: 80%, 8/10; MST>10; SAP-treated wt: 70%, 7/10; MST>10). Opsonisation with murine SAP normalized the inflammatory response (at 16h), such as TNF-α (P=0.007) (Fig. 5b) and MPO (P=0.0005) (Fig. 5c) levels and neutrophil number (P=0.02) (Fig. 5d) in BALFs, as well as it rescued or increased *in vitro* C3 deposition on *A. fumigatus* conidia after incubation with both *Apcs*^{*-*/*-*} and wt plasma (Fig. 6a). The decreased C5a formation in the presence of *Apcs*^{*-*/*-*} plasma (1min, 8.5±2.1 vs. 13.0±4.5ng/ml; 5min, 14.2±4.3 vs. 35.7±6.9ng/ml, P=0.02; 10min, 26.5±2.2 vs. 53.2±7.5ng/ml, P=0.003; 20 min, 61.5±7.8 vs. 113.8±15.5ng/ml, P=0.007, respectively *Apcs*^{*-*/*-*} vs. wt) reflecting a defective complement activation was rescued by SAP opsonisation (1min, P=0.05; 5min, P=0.04; 10min, P=0.001; 20 min, P=0.05), which also increased C5a in wt plasma (Fig.6b).

### Example 5:

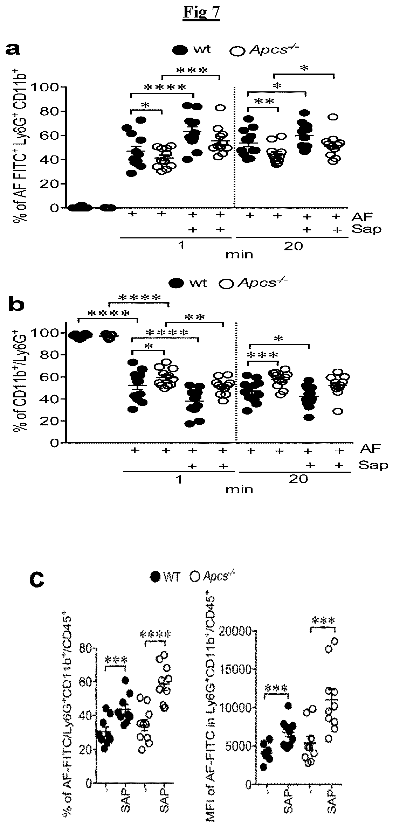

Fig. 7a and 7b depict the effect of SAP on *A. fumigatus* phagocytosis by neutrophils. a), b), FACS analysis (out of 3 performed) of phagocytosis (a) and CD11b internalization (b) in neutrophils after challenge with fluorescein-labelled AF conidia (5×10⁶/100µl of blood) opsonized or not with Sap is shown. a) neutrophil phagocytosis in whole blood of wt and *Apcs*^{*-*/*-*} mice. b) CD11b expression in neutrophils of a. a) and b) Mean±SEM; *, P<0.05; **, P<0.01; ***, P<0.005; ****, P<0.0005 (unpaired t-test).

Fig. 7c depicts the effect of human SAP on phagocytic activity. FACS analysis of neutrophil phagocytosis in blood of wt and *Apcs*^{*-*/*-*} mice after exposure with fluorescein-labelled AF conidia (5×10⁶/100µl of blood) opsonized or not with SAP. Figure refers to 2 experiments performed. ***, P<0.005; ***, P<0.0005 (unpaired t-test).

In experiments conducted in whole blood, phagocytosis by neutrophils was reduced in *Apcs*^{*-*/*-*} mice, both at 1 (47.0±4.0 vs. 41.3±2.4%; P=0.05) and 20 min (55.7±3.1 vs. 44.4±2.3%; P=0.01) after incubation with*A*. *fumigatus* conidia (Fig. 7a). Hence, SAP basal levels would be sufficient to affect conidia phagocytosis. SAP pre-opsonisation rescued the defect (1 min, P=0.002; 20 min, P=0.03) and it potentiated phagocytosis by wt neutrophils (1 min, P<0.0001; 20 min, P=0.03) (Fig. 7a). Similar results were obtained when human native SAP was used (Fig. 7c). In addition, expression of CD1 1b in wt neutrophils passed from 97.2±0.4% to 52.5±3.8% and 47.4±2.8%, respectively at 1 and 20 min after exposure to *A. fumigatus* (Fig. 7b). In neutrophils from *Apcs*^{*-*/*-*} mice, the decrease in CD11b expression was minor (basal, 97.0±0.5%; 1 min, 59.6±2.4%, P=0.05; 20 min 57.7±2.2%, P=0.005), in agreement with SAP-mediated engagement of FcyRs, which induces activation and accumulation of CD11b in the phagocytic cup for optimal phagocytosis (Lu *et al.,2012* ; Moalli *et al.,* 2010 ; Jongstra-Bilen *et al.,* 2003). SAP pre-opsonisation restored CD11b internalization and prompted it in wt (Fig. 7b). Therefore, SAP acts as opsonin for early disposal of *A. fumigatus* by neutrophils as result of enhancement of phagocytic activity.

### Example 6:

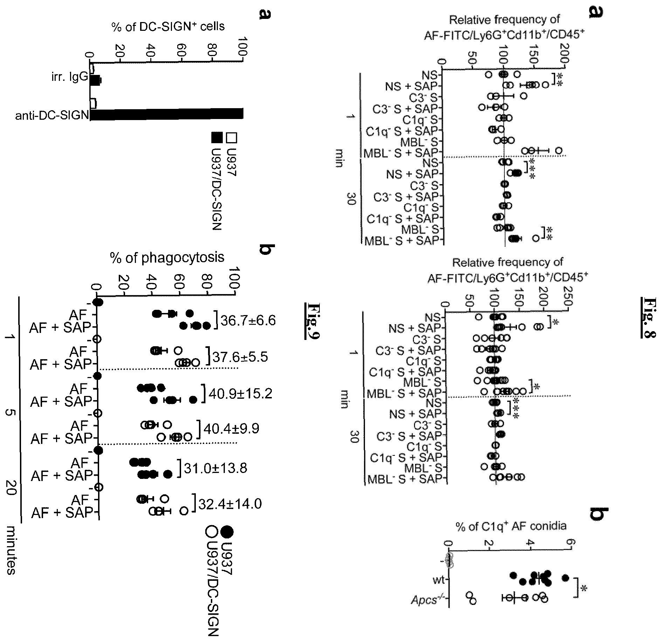

Fig. 8 depicts the initiation of classical complement activation at the bases of SAP-mediated phagocytosis. a) fluorescein-labelled AF conidia (1.6×10⁶) phagocytosis by freshly isolated human neutrophils (2×10⁵) in presence of sera depleted (⁻) from complement components and the effect of SAP opsonisation after 1 and 30 min. AF conidia (2×10⁸) opsonisation with 100µg of human native SAP. 5% (left) or 10% (right) of human sera were used. Mean±SEM; *, P<0.05; **, P<0.01; ^{∗∗∗}, P<0.005; ^{∗∗∗}, P<0.0001 (Mann-Whitney test). b) FACS analysis of C1q deposition on AF conidia (1×10⁷) in presence of plasma from wt or *Apcs*^{*-*/*-*} mice. Mean±SEM; *, P=0.05.

Classical and lectin pathways are both main initiators of complement activation against *A. fumigatus* (Rosbjerg *et al.,* 2016). Heterocomplex of mannose-binding lectin (MBL) and SAP triggers cross-activation of complement on *Candida albicans* (Ma *et al.,* 2011). Experiments of opsonophagoytosis were therefore performed in human sera deficient for different complement components to define the molecular mechanism responsible for SAP resistance to *A. fumigatus.* Phagocytosis of blood-derived human neutrophil was abolished in serum depleted for C3 (-72.0±4.7%, P<0.0001), C1q (-85.3±0.7%, P<0.0001) and MBL (-91.7±1.9%, P<0.0001) compared to normal, thus indicating importance of both classic and lectin pathways in resistance against this fungus (Rosbjerg *et al.,* 2016). In two independent experiments using 5 and 10% of sera, opsonisation of human native SAP potentiated phagocytosis in normal (5%, 37.4±10.3%, P=0.009; 10%, 18.3±2.0%, P=0.01) and MBL-depleted serum (5%, 55.5±17.0%; 10%, 19.8 ±6.1%, P=0.04), but not in those depleted for C3 and C1q (Fig. 8a), hence indicating interaction with classical complement pathway a requirement for the initiation of SAP-mediated phagocytosis. In agreement, decreased C1q deposition on *A. fumigatus* conidia was observed after incubation with serum from *Apcs*^{*-*/*-*} mice (P=0.05) ((Fig. 8b).

### Example 7:

Fig. 9 depicts how SAP effect on phagocytosis is independent from its interaction with DC-SIGN. a) detection of DC-SIGN expression in transfected U937 cells by FACS. Untransfected cells and an irrelevant IgG were used as control. Mean±SD of a quadruplicate experiment is shown. b) FACS analysis of fluorescein-labelled AF conidia (1×10⁷) phagocytosis by U937 cells (1×10⁵) overexpressing DC-SIGN. Figure shows the mean±SD percentage of SAP-mediated enhancement of phagocytosis in a quadruplicate experiment.

SAP was suggested as ligand for DC-SIGN (CD209; mouse SIGN-R1) on neutrophils and macrophages in the context of fibrosis (Cox *et al.,* 2015). Genetic variation in DC-SIGN affects susceptibility to invasive aspergillosis (Fisher *et al.,* 2017; Sainz *et al.,* 2012). Therefore, we assessed the actual involvement of DC-SIGN in SAP-mediated A. *fumigatus* phagocytosis. SAP effect was similar in a monocytic cell line stably transfected for surface expression of DC-SIGN and in control cells (Fig. 9a and b) thus suggesting no relevance of SAP and DC-SIGN interaction.

### Example 8:

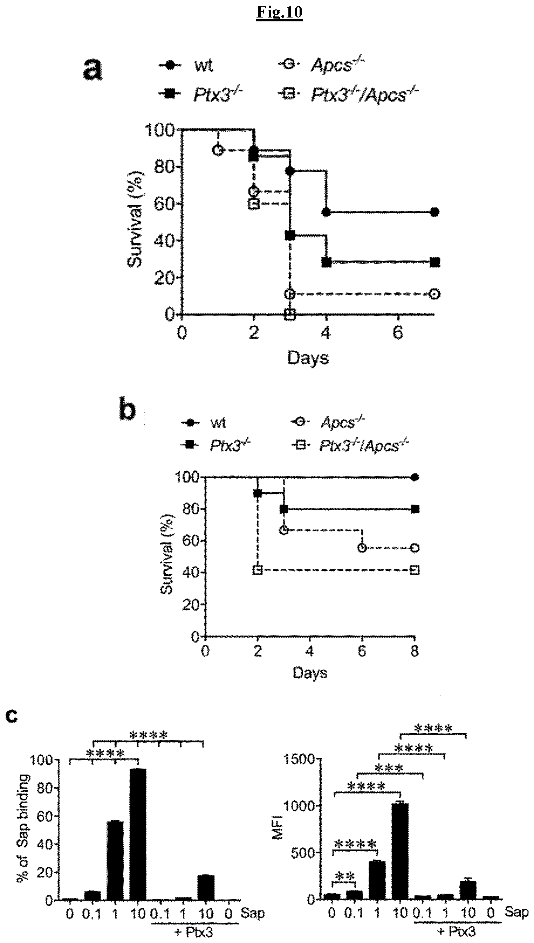

Fig. 10 depicts the susceptibility to *A. fumigatus* associated with single or double deficiency for SAP and PTX3. a and b, survival of wt and *Apcs*^{*-*/}*⁻, Ptx3*^{*-*/*-*} and *Apcs*^{*-*/}*⁻ Ptx3*^{*-*/*-*}mice after i.t. injection of 1×10⁸ (a) or 5×10⁷ (b) conidia. a, wt, n=9 (a, b); *Apcs*^{*-*/}*⁻,* n=9 (a, b); *Ptx3*^{*-*/}*⁻,* n=7 (a) or n=10 (b); *Apcs*^{*-*/}*⁻ Ptx3*^{*-*/}*⁻,* n=5 (a) or n=12 (b). a, b, P<0.05, wt vs. *Apcs*^{*-*/-}; P<0.01 wtvs. *Apcs*^{*-*/}*⁻ Ptx3*^{*-*/*-*}; P=0.06, *Apcs*^{*-*/}*⁻ Ptx3*^{*-*/*-*} vs. *Ptx3*^{*-*/*-*} (Mantel-Cox test). a) Curves of wt and *Apcs*^{*-*/*-*} refer to the same ones shown in Fig. 2a -c FACS analysis of Sap (range from 0.1 to 10 µg/ml) binding to viable AF conidia (1×10⁸) in presence of Ptx3 (50 µg/ml). An anti-Sap monoclonal antibody (non-reactive to Ptx3) was used. Mean±SD of a triplicate. **, P<0.01; ***, P<0.005; ****, P<0.0001 (unpaired t-test).

Possible functional redundancy between pentraxins of systemic and local production was evaluated in mice with single or double deficiency for SAP and PTX3. As expected, an increased susceptibility to infection was observed both in *Apcs*^{*-*/*-*} (8/9 non-survived mice at MST of 3 days; P=0.02) and *Ptx3*^{*-*/*-*} (4/7, at MST of 3 days) mice compared to wt (2/9, on day 3) after injection with 1×10⁸ of conidia (Fig. 10a). In doubly deficient mice for SAP and PTX3 mortality was further augmented (5/5 non-survived mice at MST of 3 days; P=0.008 vs. wt; P=0.06 vs. *Ptx3*^{*-*/*-*})*.* Actually, 0% of Apcs^{*-*/}*⁻ Ptx3*^{*-*/*-*} mice survived at the end of the experiments compared to 11.1% (1/9), 28.6% (2/7) and 55.6% (5/9) respectively for *Apcs*^{*-*/}*⁻, Ptx3*^{*-*/*-*} and wt mice (Fig. 10a). As shown in a representative experiment (Fig.10b), similar results were obtained when 5×10⁷ conidia were used [(non-survived mice: 4/9 (*Apcs*^{*-*/*-*})*,* 2/8 (*Ptx3*^{*-*/*-*})*,* 7/12 (*Apcs*^{*-*/}*⁻ Ptx3*^{*-*/-}*),* survival, 55.6% (*Apcs*^{*-*/*-*})*,* 80% (*Ptx3*^{*-*/-}*),* 41.7% (*Apcs*^{*-*/}*⁻ Ptx3*^{*-*/-})]*.* Table 1 summarizes results obtained in the series of experiments using 5×10⁷ conidia in the four genotypes. The binding of murine SAP (range of 0.1-10µg/ml) to conidia was competed in the presence of murine PTX3 (P<0.0001) (Fig. 10c), therefore suggesting sharing between SAP and PTX3 of the same molecular target on the fungus.

**Table 1**

| Genotype | MST (day) | Dead/Total (n) | Survival (%) | P (Fischer's exact test) |
|---|---|---|---|---|
| wt | Undefined | 7/39 | 82.0% | |
| *Apcs*^{*-*/*-*} | 3 | 34/46 | 26.1% | *P*<0.0001 vs. wt |
| *Ptx3*^{*-*/*-*} | 4.5 | 13/32 | 59.4% | *P*=0.035 vs. wt |
| *Apcs*^{*-*/}*⁻ Ptx3*^{*-*/*-*} | 3 | 24/31 | 22.5% | *P*<0.0001 vs. wt |
| | | | | *P*=0.003 vs. *Ptx3*^{*-*/*-*} |
| | | | | *P*=n.s. vs. *Apcs*^{*-*/*-*} |

### Example 9:

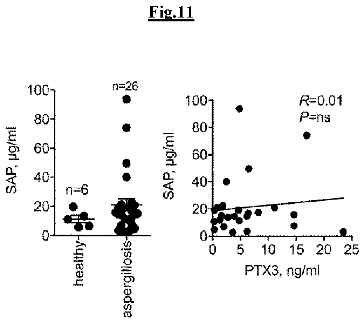

Fig. 11 depicts levels of SAP in patients affected by invasive aspergillosis.

PTX3 represents a specific marker of invasive aspergillosis (Kabbani *et al.,* 2017; Cunha *et al.,* 2014). In a cohort of 26 patients having *A. fumigatus* colonization or invasive aspergillosis median of circulating SAP (median, 15.36µg/ml; IQR, 9.93-20.94) was not significant different (P=0.25, Mann-Whitney) than in 6 healthy control subjects (median, 10.97µg/ml; IQR, 6.11-16.53) and no correlation with the circulating levels of PTX3 was observed (R=0.01) (Fig. 11), thus indicating SAP not a specific indicator of disease in humans.

### Example 10:

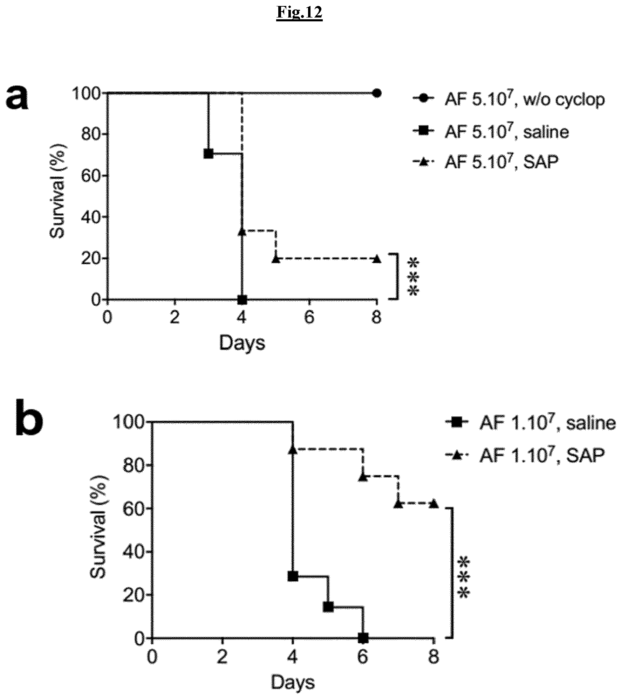

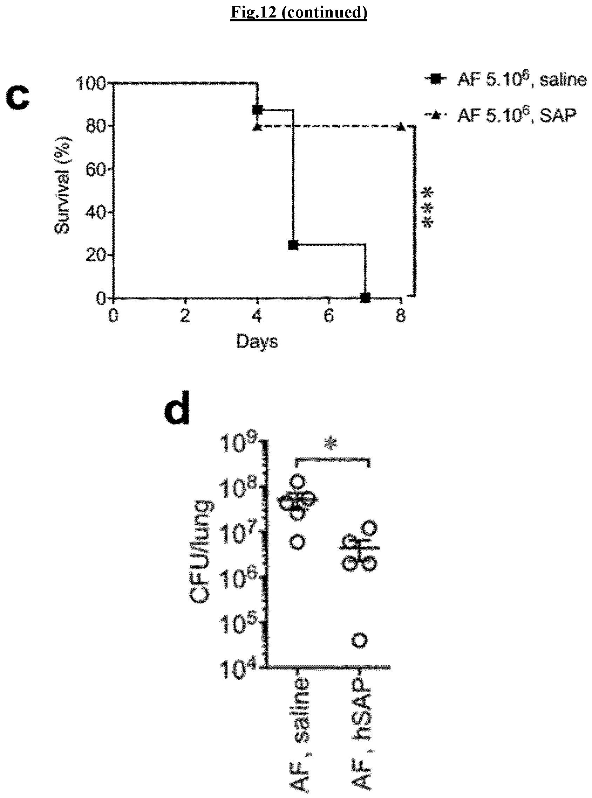

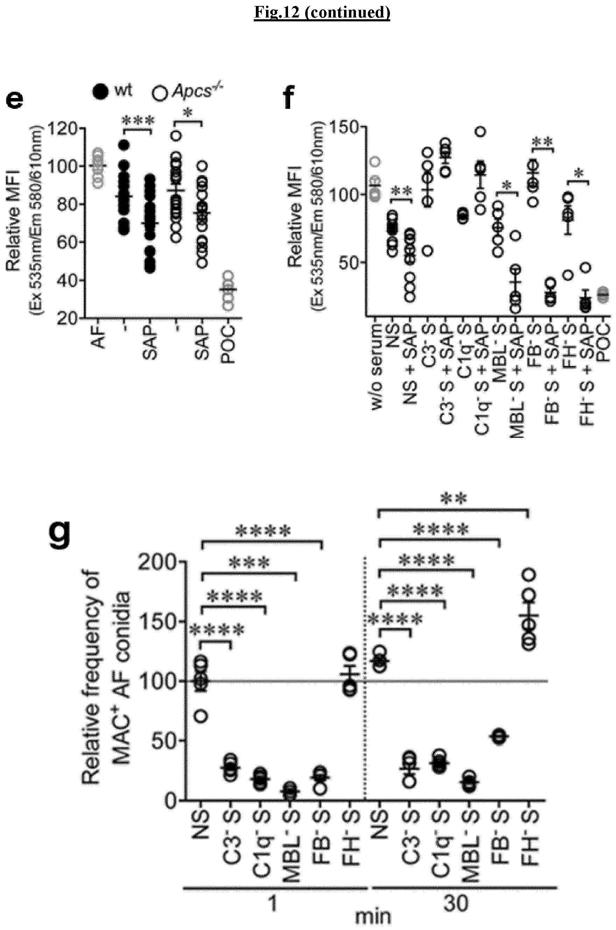

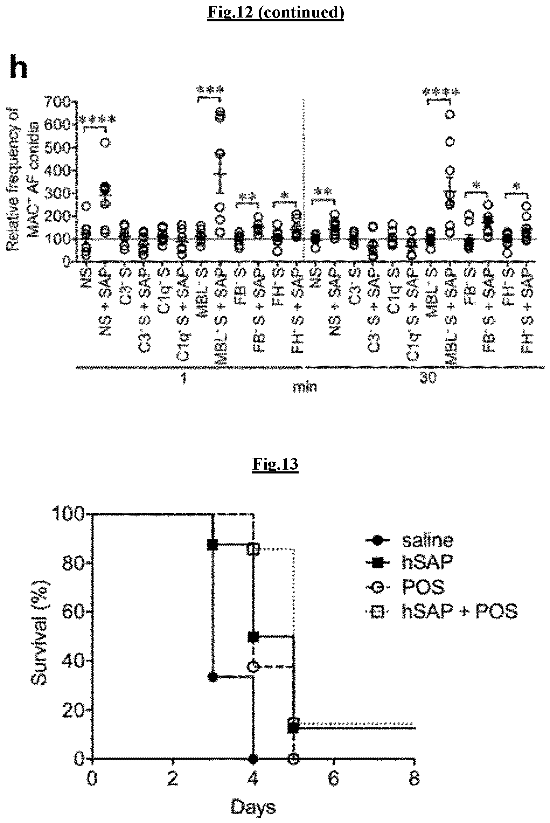

Fig. 12 depicts the therapeutic efficacy of SAP in treatment of invasive aspergillosis. a) b) c) survival of transiently immunosuppressed wt mice after injection with the indicated doses of AF conidia. The curve of untreated mice (n=3) with cyclophosphamide is also shown (a). Human SAP (4mg/Kg) was injected at 2h and 24h after infection. a) AF 5.10⁷, n=17; AF 5.10⁷ + SAP, n=15; b) AF 1.10⁷, n=7; AF 1.10⁷ + SAP, n=8; c) AF 5.10⁶, n=8; AF 5.10⁶ + SAP, n=10. ***, P<0.005 SAP-treated vs. saline (Mantel-Cox test). One-way ANOVA, P<0.0001. d) lung CFU in mice treated with cyclophosphamide and infected with 1.10⁷ AF conidia. *, P<0.05 (Mann-Whitney test). e) quantitative analysis of AF viability performed using a resazurin-based assay. Plasma (30%) from wt and *Apcs*^{*-*/*-*} mice were incubated (1 min) with 1.5×10⁵ AF conidia pre-opsonized or not with SAP. Results are mean±SD of red fluorescence (excitation/emission 535/580-610nm) intensity of the resazurin once reduced to resorufin within viable cells. Effect of Posaconazole (POC; 1 µM) exposure is also shown. Results are mean±SEM relative to the condition without serum (grey symbols) of two experiments performed with n=10 mice/group. Similar results were obtained using 10% of serum and at 30 min. ***, P<0.005; *, P<0.05 (unpaired t-test). f) SAP effect on microbicidal activity is dependent on classical complement activation. AF viability assay performed in sera depleted from complement components. Figure summarizes one experiment out of 5 also conducted with serum 10%. Mean±SD. *, P<0.05; **, P<0.01 (unpaired t-test). g) and h) FACS analysis of MAC deposition on AF conidia (1×10⁷) in presence of sera depleted from complement components (e) and the effect of SAP (f). Mean±SEM of 2 experiments performed out of 4. e, f, *, P<0.05; **, P<0.01; ***, P<0.005; ****, P<0.001; (Mann-Whitney test).

Fig. 13 depicts the therapeutic efficacy of SAP in treatment of invasive aspergillosis alone or in combination with posoconazole. Survival of transiently cyclophosphamide-immunosuppressed mice after injection with 5×10⁷ conidia. Human SAP (4 mg/Kg) and Posoconazole (POS; 1.6 mg/Kg) were injected at 16h and 40h after infection. AF 5.10⁷ + saline, n=9; AF 5.10⁷ + SAP, n=8; AF 5.10⁷ + POS, n=9; AF 5.10⁷ + SAP + POS, n=8. P<0.01, saline vs. hSAP; P<0.005, saline vs. POS; P<0.0005, saline vs. hSAP + POS; P<0.05, POS vs. hSAP + POS; (Mantel-Cox test).

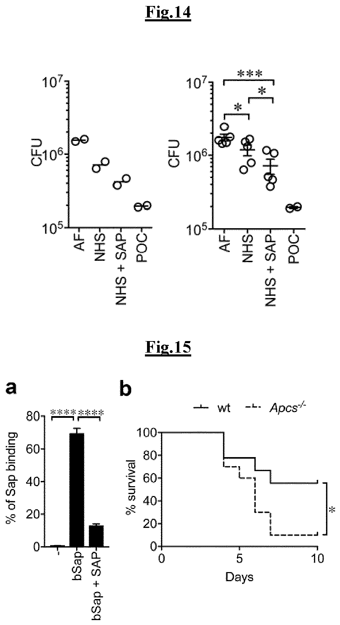

Fig. 14 depicts the determination of SAP-mediated killing of *A. fumigatus* conidia. Assessment as CFU count of the AF viability performed in normal human serum (10%) with or without SAP opsonisation. Two independent experiments are shown. *, P<0.05; ***, P<0.005 (unpaired t-test).

The most important risk factor for invasive aspergillosis is represented by neutropenia and monocytopenia that occur in immune-compromised patients (Cunha *et al.,* 2014). A potential therapeutic effect of SAP was therefore determined in transiently myelosuppressed mice. Dosage of *A. fumigatus* conidia was newly optimized in mice after 2-day treatment with cyclophosphamide (150 mg/Kg). Human native SAP (4 mg/Kg) was intraperitoneally (i.p.) injected at 2 and 24h after infection, a dose selected on the bases of SAP circulating levels upon exposure *A. fumigatus* (range of 19.2-31.7 µg/ml). Immune-compromised mice did not survive to infection with 5×10⁷ (17/17; MST 4 days) (Fig. 12a), 1×10⁷ (7/7; MST 4 days) (Fig. 12b) and 5×10⁶ (8/8; MST 5 days) (Fig. 12c) conidia . SAP protected immune-compromised mice increasing survival respectively to 20% (5×10⁷; 12/15 non-survived mice, MST 4; P=0.002) (Fig. 12a), 62.5% (1×10⁷; 3/8, MST >10; P=0.001) (Fig. 12b) and 80% (5×10⁶; 2/10, MST >10; P=0.004) (Fig. 12c). A 12-fold reduction in fungal burden was observed in lung of SAP-treated mice (16 h) after infection with 1×10⁷ conidia (median 2.0×10⁶ CFU, IQR 9.0×10⁶-8.0×10⁶ vs. 4.4×10⁷ CFU, IQR 9.1×10⁷-7.5×10⁷; P=0.02) (Fig. 12d). Similar effect was obtained in a different experiment where immunosuppressed mice were treated with human SAP (4mg/Kg) and the antifungal Posaconazole (POC; 1.6mg/Kg) 16h from infection with 5×10⁷ conidia (Fig. 13).

Results prompted us to evaluate a cell-independent SAP effect on fungal killing. In a resazurin-based cell viability assay, pre-opsonisation of human SAP resulted in increase of microbicidal activity observed in plasma of wt (P=0.004) and *Apcs*^{*-*/*-*} (P=0.02) mice (1 min). POC was used as antifungal control (Fig. 12e). The effect was abolished in human sera depleted of C3 or C1q (Fig. 12f). Assembly of the membrane attack complex (MAC; C5b-C9) on the surface of *A. fumigatus* conidia was decreased in sera depleted for C3 (1 min, -72.2±4.8%; 30 min, -73.0±10.1%; Mean±SD; P<0.0001), C1q (1 min, -81.8±3.6%; 30 min, -68.5±4.0%; P<0.0001), MBL (1 min, -92.2±2.8%, P<0.001; 30 min, -84.5±3.0%, P<0.0001) and FB (1 min, -80.6+5.2%; 30 min, 46.4+1.0%; P<0.0001) and increased in FH-depleted serum (1 min, +5.6±15.8%; 30 min, +55.0±24.9%, P<0.005) (Fig. 12g). Pre-opsonisation with human SAP enhanced MAC formation on conidia both at 1 min (P=0.001) and at 30 min (P=0.008) in the presence of normal serum and in those depleted of MBL, FB and FH, but not in serum depleted for C1q (Fig. 12h), therefore suggesting a role of SAP to direct pathogen destruction through classical complement activation in conditions of deficiency of immune competent cells. In some experiments, the actual fungal killing was ascertained as also CFU count (Fig. 14).

### Example 11:

Fig. 15 depicts the susceptibility of SAP-deficient mice to A. *flavus.* a) FACS analysis of binding of biotin-conjugated (b-) murine SAP (Sap; 10µg/ml) to conidia of A. *flavus* (1×10⁸). Human SAP (50µg/ml) was also used to compete binding of Sap. Mean±SD; ****, P<0.0001 (unpaired t-test). b) susceptibility of SAP-deficient mice to *A. flavus* infection. Survival of wt and *Apcs*^{*-*/*-*} mice after i.t. injection of 5×10⁷ conidia; wt, n=9; *Apcs*^{*-*/-,} n=10. *, P=0.05 (Log-rank; Mantel-Cox test).

### Example 12:

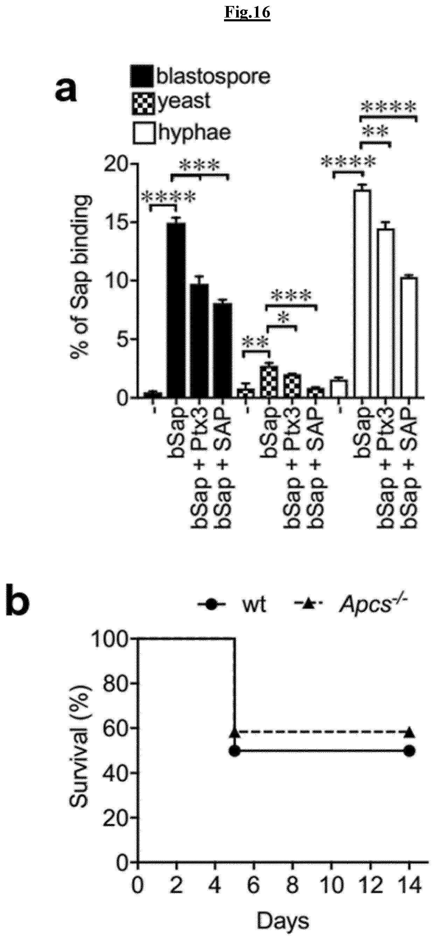

Fig. 16 depicts the binding of Sap on Candida. a) FACS analysis of binding of biotin-conjugated (b-) murine SAP (Sap; 10µg/ml) to bastospore, yeast and hyphae of Candida *albicans* (1×10⁸). Human SAP (50µg/ml) was also used to compete binding of Sap. Mean±SD; ****, P<0.000; ***, P<0.005; **, P<0.01; *, P<0.05; (unpaired t-test). b) Sap deficient mice do not show a different susceptibility to Candida *albicans* bloodstream infection. Survival of wt and *Apcs*^{*-*/*-*} mice after retroorbital injection of 1×10⁶ blastospores of Candida *albicans.* wt, n=10; *Apcs*^{*-*/*-,*} n=12.

Heterocomplex of mannose-binding lectin (MBL) and SAP triggers cross-activation of complement on Candida *albicans in vitro* (Ma *et al.,* 2011). Further experiments were therefore conducted to effectively evaluate *in vivo* relevance of SAP as an antifungal molecule also in Candida infections. As also reported (Ma *et al.,* 2011), recombinant murine SAP (Sap; 10 µg/ml) bound with low affinity blastospores, yeasts and hyphae of Candida *albicans.* Sap binding was however competed by human SAP (50 µg/ml) (Fig. 16a). In experimental Candida *albicans* bloodstream infection, SAP-deficiency was not associated to a different susceptibility (Fig. 16b).

### Materials & Methods

*Animals.* Wild-type C57BL/6J mice between 8 and 10 weeks of age were purchased from Charles River Laboratories (Calco, Como, Italy). *Apcs*^{*-*/*-*} mice were kindly provided by Professor Marina Botto (Imperial College, London, UK). *Ptx3*^{*-*/*-*} mice were generated as described²⁶. *Apcs*^{*-*/}*⁻ Ptx3*^{*-*/*-*} mice were generated by crossing mice with single deficiency. All mice were used on a C57BL/6J genetic background. Mice were housed and bred in the SPF animal facility of Humanitas Clinical and Research Center in individually ventilated cages. Procedures involving animals and their care were conformed to protocols approved by the Clinical and Research Institute Humanitas (Rozzano, Milan, Italy) in compliance with national (4D.L. N.116, G.U., suppl. 40, 18-2-1992) and international law and policies (EEC Council Directive 2010/63/EU, OJ L 276/33, 22-09-2010; National Institutes of Health Guide for the Care and Use of Laboratory Animals, US National Research Council, 2011). The study was approved by the Italian Ministry of Health (approval n. 71/2012-B, issued on the 09/03/2012 and 44/2015-PR issued 28/01/2015). All efforts were made to minimize the number of animals used and their suffering.

*Invasive pulmonary aspergillosis.* Haematological samples from patients affected by fungal infection caused by *Aspergillus spp.* were provided by Prof. van de Veerdonk (Radboud University Medical Center, Nijmegen, The Netherlands). For patient studies, drawing of blood samples from patients was approved by the local ethical board at the Radboud University Nijmegen (Arnhem-Nijmegen Medical Ethical Committee).

A clinical strain of *A. fumigatus* was isolated from a patient with a fatal case of pulmonary aspergillosis was kindly provided by Dr. Giovanni Salvatori (Sigma-tau, Rome, Italy) (Pepys *et al.,* 2012). Aspargillus flavus (#ATCC^{®} 9643^{™}) was obtained from ATCC.

The growth and culture conditions of *A. fumigatus* and *A. Flavus* conidia were as described (Garlanda *et al.,* 2002). For intratracheal (i.t.) injection, mice were anesthetized by i.p. injection of ketamine (100 mg/Kg; i.p.) and xylazine (10 mg/Kg i.p.). After surgical exposure, a volume of 50µl PBS²⁺, pH 7.4, containing 1×10⁸ or 5×10⁷ resting conidia (>95% viable, as determined by serial dilution and plating of the inoculum on Sabouraud dextrose agar) were delivered into trachea under direct vision using a catheter connected to the outlet of a micro-syringe (Terumo, Belgium). Survival to infection was daily monitored for 10d later. Dying mice were euthanized after evaluation of the following clinical parameters: body temperature dropping, intermittent respiration, solitude presence, sphere posture, fur erection, non-responsive alertness, and inability to ascent when induced.

In experiments of *in vivo* phagocytosis, mice were i.t. injected with 5×10⁷ heat inactivated fluorescein isothiocyanate (FITC)-labelled conidia and euthanized 4h later. In rescue experiments, conidia (1×10⁹) were opsonized with murine recombinant SAP (50µg/ml; R&D Systems) for 1h at r.t. in PBS, pH 7.4, containing 0.01% (vol/vol) Tween-20^{®} (Merck-Millipore). After washing of unbound protein, a volume of 50 µl (5×10⁷ conidia) was i.t. injected.

In therapy experiments, immunosuppression was induced by i.p. injection of 150mg/Kg cyclophosphamide (150µl per mouse of 20 mg/ml solution) 2d before infection. Native human SAP (Merck-Millipore) was dialysed in PBS (pH 7.4) in order to eliminate sodium azide and i.p. injected at the dose of 4mg/Kg at 2 and 24h after infection or in combination with Posoconazole (POS; 1.6 mg/Kg) at 16h and 40h after infection.

*Disseminated candidiasis. Candida albicans* was provided by Professor Marina Vai (Biotechnology and Biosciences Department, Università degli Studi di Milano-Bicocca) and routinely grown at 25°C in rich medium [YEPD (yeast extract, peptone, dextrose), 1% (w/v) yeast extract, 2% (w/v) Bacto Peptone, and 2% (w/v) glucose] supplemented with uridine (50 mg/liter) as described (Santus *et al.* , 2017). For survival experiments, a colony of C. albicans was collected by a culture plate and grown under rotation for 24h at 37°C in YEPD medium, and, once centrifuged (1000 rpm for 5 min), cells were injected into the retro-orbital plexus at 1.10⁵/200 µl PBS. Survival of mice was monitored for two weeks.

*Tissue injury.* Skin wounding and chemical-induced liver injury was performed as previously described (Doni *et al.,* 2015).

*BALFs collection and analysis.* BALFs were performed with 1.5ml PBS, pH 7.4, containing protease inhibitors (Complete tablets, Roche Diagnostic; PMSF, Sigma-Aldrich) and 10mM EDTA (Sigma-Aldrich) with a 22-gauge venous catheter. BALFs were centrifuged, and supernatants were collected for quantification of total protein content with Bradford's assay (Bio-RAD) and cytokines as described below. After erythrocyte lysis with ACK solution (pH 7.2; NH₄Cl 0.15 M, KHCO₃ 10 mM, EDTA 0.1 mM), cells were resuspended in PBS, pH 7.4, containing 10mM EDTA and 1% heat-inactivated fetal bovine serum (FCS; Sigma-Aldrich), stained with live and death dye (ThermoFisher Scientific-Molecular Probes) and analysed by BD FACS Canto^{™} II Flow Cytometer (BD Biosciences) with the following specific antibodies: peridinin chlorophyll protein complex (PerCP)- or brilliant violet (BV) 650-labelled anti-CD45 (#30-F11, IgG_{2b}; 4µg/ml); FITC- or phycoerythrin (PE)-CF594-labelled anti-Ly6G (#1A8, IgG₂ₐ; 4 µg/ml); allophycocyanin (APC)- or BV421-labelled anti-CD11b (#M1/70, RUO, IgG_{2b}; 1 µg/ml) (all from BD Biosciences).

*Lung homogenates and analysis.* Lungs were removed 16h after infection and homogenized in 1ml PBS, pH 7.4, containing 0.01% (vol/vol) Tween-20^{®} (Merck-Millipore) and protease inhibitors. Samples were serially diluted 1:10 in PBS and plated on Sabouraud dextrose agar for blinded CFU counting. For lysate preparation, lungs were collected at 4h and homogenized in 50mM Tris-HCl, pH 7.5, containing 2mM EGTA, 1mM PMSF, 1% Triton X-100 (all from Sigma-Aldrich), and complete protease inhibitor cocktail. Total proteins were measured by DC Protein Assay, according to manufacturer's instructions (Bio-Rad Laboratories). Western blot analysis for C3 was performed after loading 10µg of lung protein extracts on SDS-PAGE. The goat polyclonal anti-C3 (1:3000; Merck-Millipore) and HRP-conjugated donkey anti-goat IgG (1:5000; R&D Systems) were used. The monoclonal anti-vinculin (0.5 µg/ml; hVIN-1; Sigma-Aldrich) was used as loading control. C3d bands were quantified by Fiji-ImageJ (NIH, Bethesda USA) as a ratio of mean grey intensity values of each protein relative to vinculin bands.

*Cells and in vitro phagocytic activity.* Phagocytosis assay in whole blood of *A. fumigatus* conidia by mouse and human neutrophils was performed as described (Moalli *et al.,* 2010). Briefly, conidia (1×10⁹) were labelled (1h, r.t.) with FITC (Sigma-Aldrich; 5 mg/ml in DMSO), and eventually opsonized (1h, r.t) with murine SAP (100 µg/ml; 1.1 µM) and PTX3 (50 µg/ml; 1.1µM). An amount of 1×10⁷ FITC-conidia were added to 200 µl of mouse whole blood (collected with heparin) and incubated for 1, 5, 10, 20 or 30 min at 37°C in an orbital shaker. Samples were immediately placed on ice and ACK lysis solution was added to lyse erythrocytes. Murine neutrophils were analysed by BD FACS Canto^{™} II Flow Cytometer (BD Biosciences) as previously described, and frequency and/or mean fluorescence intensity (MFI) of FITC⁺ neutrophils and CD11b expression were reported.

Human neutrophils were isolated from fresh whole blood of healthy volunteers through separation from erythrocytes by 3% dextran (GE Healthcare Life Sciences) density gradient sedimentation followed by Ficoll-Paque PLUS (GE Healthcare Life Sciences) and 62% Percoll^{®} (Sigma-Aldrich) centrifugation as previously described (Moalli *et al.,* 2010). Purity, determined by FACS analysis on forward scatter/side scatter parameters, was routinely >98%. 1×10⁵ neutrophils were incubated for 1 and 30min at 37°C in 50 µl RPMI-1640 medium with 5 and 10% normal human serum (NS) or complement depleted sera and 1×10⁵ FITC-labelledA. fumigatus conidia. Cells were transferred on ice and, after washing with PBS, pH 7.4, containing 10mM EDTA and 0.2% bovine serum albumin (BSA; Sigma-Aldrich), FITC fluorescence in neutrophils (CD45-positive cells, neutrophils defined as FSC-A^{high}/SSC-A^{high}) was analysed by BD FACS Canto^{™} II Flow Cytometer (BD Biosciences). NS and sera depleted for C3, C1q, MBL, FB and FH were all obtained from CompTech (Complement Technology, Inc., USA).

U937 cell lines [control (ATCC^{®} CRL-1593.2^{™}) and transduced with human DC-SIGN (ATCC^{®} CRL-3253^{™})] were cultured in RPMI-1640 medium containing 5% FCS, 2 mM L-glutamine, 0.1 mM non-essential amino acids (all from Lonza-BioWhittaker^{™}) and 0.05 mM 2-mercaptoethanol (BIO-RAD). DC-SIGN expression was ascertained by FACS using a rabbit polyclonal Ab (#ab5715, 5µg/ml; AbCam, UK) and an Alexa Fluor^{®} 488-conjugated goat anti rabbit (2 µg/ml; ThermoFisher-Molecular Probes). Phagocytosis of FITC-labelled *A. fumigatus* conidia (1×10⁷) by U937 cells (1×10⁵) was performed as above described above.

*Complement deposition on Aspergillus fumigatus.* A volume of 10 µl PBS, pH 7.4, containing 1×10⁷ conidia eventually opsonized with murine SAP (100 µg/ml per 1×10⁹ conidia, 1h at r.t.) was incubated (37°C) in round bottom wells of Corning^{®} 96-well polypropylene microplates for the indicated time points with 20 µl mouse plasma-heparin or 20 µl human NS and complement depleted sera (diluted in PBS at 10% and 30%). Complement deposition was blocked by addition of EDTA (10 mM final concentration) and by cooling in ice. After centrifugation (2000 rpm, 10 min at 4°C), when indicated supernatant was collected for C5a measurement by ELISA. Conidia were washed and incubated (1h, at 4°C) with PBS, pH 7.4, 2mM EDTA, 1%BSA containing the following primary antibodies: goat anti-C3 and activation fragments (1:5000; Merck-Millipore); rat anti-C1q (IgG₁, #7H8, 1 µg/ml; HyCult^{®} Biotech, Netherlands); rat anti-MBL (IgG₂ₐ, #8G6, 1µg/ml; HyCult^{®} Biotech, Netherlands) or rabbit anti-MBL (2µg/ml; AbCam, UK); rabbit anti- C5b-C9 (MAC) (1:2000; Complement Technology, Inc.); or correspondent irrelevant IgGs. Conidia were then incubated (1h, at 4°C) with Alexa Fluor^{®} 488 and 647-conjugated species-specific cross-adsorbed detection antibodies (2µg/ml; ThermoFisher Scientific-Molecular Probes) and analysed by BD FACS Canto^{™} II Flow Cytometer (BD Biosciences) using forward and side scatter parameters to gate on at least 8,000 conidia. After each step, conidia were extensively washed with PBS, pH 7.4, 2 mM EDTA, 1%BSA. Results were expressed as frequency of conidia showing fluorescence compared with irrelevant controls and as geometric conidia MFI.

*Fungal viability assay.* Effect of SAP on *A. fumigatus* killing was evaluated by a resazurin-based cell viability assay as described (Mantovani *et al.,* 2010). A volume of 10µl PBS, pH 7.4, containing 1.5×10⁵ conidia eventually opsonized with human SAP (100µg/ml per 1×10⁹ conidia, 1h at r.t.) was placed into sterile round bottom Corning^{®} 96-well polypropylene microplate and incubated for 1 and 30min at 37°C with 20µl of 10 and 30% plasma-heparin from wt and *Apcs*^{*-*/*-*} mice or human serum and different complement component-depleted sera. After incubation, plates were immediately cooled on ice and cold-centrifuged (2000rpm, 10min at 4°C), and then supernatant removed. Conidia not incubated with plasma or serum were used as a negative control. Conidia treated with the fungicide drug Posaconazole (POC; 1µM) were considered as positive control in the assay. Preparation of AlamarBlue^{™} Cell Viability Reagent and test was performed according with manufacturer's instructions (ThermoFisher Scientific-Invitrogen). A volume of 100µl AlamarBlue^{™} solution (10 µl of AlamarBlue^{™} reagent and 90µl of Sabouraud dextrose broth) was added to each well. After 17h reaction at 37°C, fluorescence (excitation/emission at ≈530-560/590nm) intensity was measured by microplate reader Synergy^{™} H4 (BioTek, France). Results represent ratio of fluorescence intensity values relative to those measured in negative controls. The actual killing of fungi was controlled as CFU count as previously described.

*Proteins.* A recombinant murine SAP from mouse myeloma cell line NSO was used (R&D Systems). Native SAP from human serum was purchased by Merck-Millipore. Recombinant murine PTX3 was purified from Chinese hamster ovary cells constitutively expressing the proteins, as described previously (Moalli *et al.,* 2010). Purity of the recombinant protein was assessed by SDS-PAGE followed by silver staining. Recombinant PTX3 contained <0.125 endotoxin units/ml as checked by the Limulus amebocyte lysate assay (BioWhittaker^{®}, Inc.). Blood was collected with heparin from the cava vein of anaesthetised mice. SAP levels were measured in mouse plasma by ELISA (DuoSet ELISA; R&D Systems). Murine TNF-α, CCL2, MPO were measured by ELISA (DuoSet ELISA; R&D Systems). Murine C5a was measured either in plasma-heparin or in BALFs previously stored at -80°C by DuoSet ELISA (R&D Systems) maintaining EDTA (10 mM) throughout the assay in order to stop the activation of the complement cascade.

*Binding of SAP.* Conidia of *A. fumigatus* and *A. flavus* (1×10⁸ CFU) were cultured 4 and 16h under static condition in Sabouraud medium to respectively allow conidia swelling and germination. Blastospores and yeast of *Candida albicans* were prepared as previously described (Santus *et al.,* 2017). Yeast (8×10⁶/ml) were incubated at 37°C for hyphal induction. Formation of hyphae was evaluated under a microscope at different time points until its amount was assessed at 95%. After washing with PBS²⁺, pH 7.4, containing 0.01% (vol/vol) Tween-20^{®} (Merck-Millipore), Cells (1x10' CFU) were incubated (1h, r. t.) with biotin-labelled murine SAP (R&D Systems) at concentrations ranging from 0.1 to 10µg/ml in PBS²⁺, pH 7.4, containing 2% BSA (Sigma-Aldrich). In competition experiments, human SAP or CRP (50µg/ml; Merck-Millipore) or murine PTX3 (50µg/ml) were further added. After extensive washing, samples were incubated (30 min, r. t.) with Alexa Fluor^{®} 647-conjugated streptavidin and binding was evaluated by FACS as frequency and MFI and visualized by confocal microscopy as described. In some experiments, a rat monoclonal anti-SAP (IgG2a, #273902; 5 µg/ml; R&D Systems) was also used.

*Microscopy.* 5-µm cryostat sections of mouse lungs were incubated in 5% of normal donkey (Sigma-Aldrich) serum, 2% BSA (Sigma-Aldrich), 0.1% Triton X-100 (Sigma-Aldrich) in PBS²⁺, pH 7.4, (blocking buffer) for 1 h at room temperature. Sections were incubated with the following primary antibodies diluted in blocking buffer for 2h at r. t.: rabbit polyclonal anti-SAP (1:500; Merck-Millipore); goat polyclonal anti-PTX3 (2µg/ml; R&D Systems); rat monoclonal anti-C3 (C3b/iC3b/C3d) (IgG2a, #11H9; 5 µg/ml; Hycult^{®} Biotech). Sections were then incubated for 1 h with Dylight^{®} and Alexa Fluor^{®} (488, 568 and 647)-conjugated species-specific cross-adsorbed detection antibodies (ThermoFisher Scientific-Molecular Probes). For DNA detection, DAPI (300 nM; ThermoFisher Scientific-Molecular Probes) was used. After each step, sections were washed with PBS²⁺, pH 7.4, containing 0.01% (vol/vol) Tween-20^{®} (Merck-Millipore). Correspondent IgG isotype controls were used. Sections were mounted with the antifade medium FluorPreserve^{®} Reagent (Merck-Millipore) and analysed in a sequential scanning mode with a Leica TCS SP8 confocal microscope at Airy Unit 1 with an oil immersion lens 63X (N.A. 1.4). Images of SAP binding to resting or germinated conidia were obtained after z-stack acquisition using same instrument parameters and image deconvolution by Huygens Professional software (Scientific Volume Imaging B.V.) and presented as medium intensity projection (MIP).

*Statistic.* Student's t-tests were performed after data were confirmed to fulfil the criteria of normal distribution and equal variance. Otherwise Mann-Whitney test was applied. Log-rank (Mantel-Cox) test was performed for comparison of survival curves. Ordinary one-way Anova was performed for curve multiple comparisons. Statistical significance of multivariate frequency distribution between groups was also analysed by Fisher's Exact test. Analyses were performed with GraphPad Prism 6 software.

### REFERENCES

An, J.H. et al. Human SAP is a novel peptidoglycan recognition protein that induces complement-independent phagocytosis of Staphylococcus aureus. J Immunol 191, 3319-3327 (2013).

Andersen, O. et al. Serum amyloid P component binds to influenza A virus haemagglutinin and inhibits the virus infection in vitro. Scandinavian journal of immunology 46, 331-337 (1997).

Balmer, P., McMonagle, F., Alexander, J. & Stephen Phillips, R. Experimental erythrocytic malaria infection induces elevated serum amyloid P production in mice. Immunology letters 72, 147-152 (2000).

Bharadwaj, D., Mold, C., Markham, E. & Du Clos, T.W. Serum amyloid P component binds to Fc gamma receptors and opsonizes particles for phagocytosis. J Immunol 166, 6735-6741 (2001).

Bottazzi et al. , J Biol Chem. 1997 Dec 26;272(52):32817-23.

Bottazzi, B., Doni, A., Garlanda, C. & Mantovani, A. An integrated view of humoral innate immunity: pentraxins as a paradigm. Annual review of immunology 28, 157-183 (2010).

Bottazzi, B. et al. The pentraxins PTX3 and SAP in innate immunity, regulation of inflammation and tissue remodelling. Journal of hepatology 64, 1416-1427 (2016).

Casas, J.P., Shah, T., Hingorani, A.D., Danesh, J. & Pepys, M.B. C-reactive protein and coronary heart disease: a critical review. Journal of internal medicine 264, 295-314 (2008).

Cox, N., Pilling, D. & Gomer, R.H. Serum amyloid P: a systemic regulator of the innate immune response. Journal of leukocyte biology 96, 739-743 (2014).

Cox, N., Pilling, D. & Gomer, R.H. DC-SIGN activation mediates the differential effects of SAP and CRP on the innate immune system and inhibits fibrosis in mice. Proceedings of the National Academy of Sciences of the United States of America 112, 8385-8390 (2015).

Cuello et al., Mol Cell Proteomics, 2014 Oct;13(10):2545-57. doi: 10.1074/mcp.M114.039446

Cunha, C. et al. Genetic PTX3 deficiency and aspergillosis in stem-cell transplantation. The New England journal of medicine 370, 421-432 (2014).

Cunha, C. et al. PTX3-Based Genetic Testing for Risk of Aspergillosis After Lung Transplant. Clinical infectious diseases : an official publication of the Infectious Diseases Society of America 61, 1893-1894 (2015).

de Haas, C.J. et al. Serum amyloid P component bound to gram-negative bacteria prevents lipopolysaccharide-mediated classical pathway complement activation. Infection and immunity 68, 1753-1759 (2000).

Doni, A. et al. Interactions of the humoral pattern recognition molecule PTX3 with the complement system. Immunobiology 217, 1122-1128 (2012).

Doni, A. et al. An acidic microenvironment sets the humoral pattern recognition molecule PTX3 in a tissue repair mode. The Journal of experimental medicine 212, 905-925 (2015).

Du Clos, T.W. & Mold, C. Pentraxins (CRP, SAP) in the process of complement activation and clearance of apoptotic bodies through Fcgamma receptors. Current opinion in organ transplantation 16, 15-20 (2011).

Duffield and Lupher, Drug News & Perspectives 2010, 23(5):305- 315

Emsley, J. et al. Structure of pentameric human serum amyloid P component. Nature 367, 338-345 (1994).

Fisher, C.E. et al. Validation of single nucleotide polymorphisms in invasive aspergillosis following hematopoietic cell transplantation. Blood 129, 2693-2701 (2017).

Fujita, T. Evolution of the lectin-complement pathway and its role in innate immunity. Nature reviews. Immunology 2, 346-353 (2002).

Garcia-Sherman, M.C., Lundberg, T., Sobonya, R.E., Lipke, P.N. & Klotz, S.A. A unique biofilm in human deep mycoses: fungal amyloid is bound by host serum amyloid P component. NPJ biofilms and microbiomes 1 (2015).

Garlanda, C. et al. Non-redundant role of the long pentraxin PTX3 in anti-fungal innate immune response. Nature 420, 182-186 (2002).

Garlanda, C., Bottazzi, B., Bastone, A. & Mantovani, A. Pentraxins at the crossroads between innate immunity, inflammation, matrix deposition, and female fertility. Annual review of immunology 23, 337-366 (2005).

Gazendam, R.P., van de Geer, A., Roos, D., van den Berg, T.K. & Kuijpers, T.W. How neutrophils kill fungi. Immunological reviews 273, 299-311 (2016).

Genster, N. et al. Lessons learned from mice deficient in lectin complement pathway molecules. Molecular immunology 61, 59-68 (2014).

Gilchrist, K.B., Garcia, M.C., Sobonya, R., Lipke, P.N. & Klotz, S.A. New features of invasive candidiasis in humans: amyloid formation by fungi and deposition of serum amyloid P component by the host. The Journal of infectious diseases 206, 1473-1478 (2012).

Gilchrist et al., J Infect Dis. 2012 Nov;206(9):1473-8. doi: 10.1093/infdis/jis464.

Hajishengallis, G. & Lambris, J.D. Crosstalk pathways between Toll-like receptors and the complement system. Trends in immunology 31, 154-163 (2010).

Herbert, J. et al. Influenza virus infection is not affected by serum amyloid P component. Mol Med 8, 9-15 (2002).

Hind, C.R., Collins, P.M., Baltz, M.L. & Pepys, M.B. Human serum amyloid P component, a circulating lectin with specificity for the cyclic 4,6-pyruvate acetal of galactose. Interactions with various bacteria. The Biochemical journal 225, 107-111 (1985).

Holmskov, U., Thiel, S. & Jensenius, J.C. Collections and ficolins: humoral lectins of the innate immune defense. Annual review of immunology 21, 547-578 (2003).

Inforzato et al., Biochemistry 2006 Sep 26;45(38):11540-51.

Inohara, Chamaillard, McDonald, C. & Nunez, G. NOD-LRR proteins: role in host-microbial interactions and inflammatory disease. Annual review of biochemistry 74, 355-383 (2005).

Jaillon, S. et al. The humoral pattern recognition receptor PTX3 is stored in neutrophil granules and localizes in extracellular traps. The Journal of experimental medicine 204, 793-804 (2007).

Jaillon, S. et al. The humoral pattern recognition molecule PTX3 is a key component of innate immunity against urinary tract infection. Immunity 40, 621-632 (2014).

Jeannin, P. et al. Complexity and complementarity of outer membrane protein A recognition by cellular and humoral innate immunity receptors. Immunity 22, 551-560 (2005).

Job, E.R. et al. Serum amyloid P is a sialylated glycoprotein inhibitor of influenza A viruses. PloS one 8, e59623 (2013).

Jongstra-Bilen, J., Harrison, R. & Grinstein, S. Fcgamma-receptors induce Mac-1 (CD11b/CD18) mobilization and accumulation in the phagocytic cup for optimal phagocytosis. The Journal of biological chemistry 278, 45720-45729 (2003).

Kabbani, D. et al. Pentraxin 3 levels in bronchoalveolar lavage fluid of lung transplant recipients with invasive aspergillosis. J Heart Lung Transplant 36, 973-979 (2017).

Kaur, S. & Singh, P.P. Serum amyloid P-component-mediated inhibition of the uptake of Mycobacterium tuberculosis by macrophages, in vitro. Scandinavian journal of immunology 59, 425-431 (2004).

Kiernan et al. Proteomics. 2004 Jun;4(6):1825-9

Klotz, S.A., Sobonya, R.E., Lipke, P.N. & Garcia-Sherman, M.C. Serum Amyloid P Component and Systemic Fungal Infection: Does It Protect the Host or Is It a Trojan Horse? Open forum infectious diseases 3, ofw166 (2016).

Lionakis, M.S. & Levitz, S.M. Host Control of Fungal Infections: Lessons from Basic Studies and Human Cohorts. Annual review of immunology 36, 157-191 (2018).

Lu, J. et al. Structural recognition and functional activation of FcgammaR by innate pentraxins. Nature 456, 989-992 (2008).

Lu, J., Marjon, K.D., Mold, C., Du Clos, T.W. & Sun, P.D. Pentraxins and Fc receptors. Immunological reviews 250, 230-238 (2012).

Ma, Y.J. et al. Synergy between ficolin-2 and pentraxin 3 boosts innate immune recognition and complement deposition. The Journal of biological chemistry 284, 28263-28275 (2009).

Ma, Y.J. et al. Heterocomplexes of mannose-binding lectin and the pentraxins PTX3 or serum amyloid P component trigger cross-activation of the complement system. The Journal of biological chemistry 286, 3405-3417 (2011).

Ma, Y.J., Lee, B.L. & Garred, P. An overview of the synergy and crosstalk between pentraxins and collectins/ficolins: their functional relevance in complement activation. Experimental & molecular medicine 49, e320 (2017).

Magrini, E., Mantovani, A. & Garlanda, C. The Dual Complexity of PTX3 in Health and Disease: A Balancing Act? Trends in molecular medicine 22, 497-510 (2016).

Mantovani, A. Molecular pathways linking inflammation and cancer. Current molecular medicine 10, 369-373 (2010).

Mantovani, A. et al. The long pentraxin PTX3: a paradigm for humoral pattern recognition molecules. Annals of the New York Academy of Sciences 1285, 1-14 (2013).

Mantovani, A., Garlanda, C., Doni, A. & Bottazzi, B. Pentraxins in innate immunity: from C-reactive protein to the long pentraxin PTX3. Journal of clinical immunology 28, 1-13 (2008).

Marschner et al. Front Immunol. 2018 Sep 25;9:2173. doi: 10.3389/fimmu.2018.02173

Martin, P. & Leibovich, S.J. Inflammatory cells during wound repair: the good, the bad and the ugly. Trends in cell biology 15, 599-607 (2005).

Moalli, F. et al. Role of complement and Fc{gamma} receptors in the protective activity of the long pentraxin PTX3 against Aspergillus fumigatus. Blood 116, 5170-5180 (2010).

Mold, C., Gresham, H.D. & Du Clos, T.W. Serum amyloid P component and C-reactive protein mediate phagocytosis through murine Fc gamma Rs. J Immunol 166, 1200-1205 (2001).

Moreira, A.P. et al. Serum amyloid P attenuates M2 macrophage activation and protects against fungal spore-induced allergic airway disease. The Journal of allergy and clinical immunology 126, 712-721 e717 (2010).

Ng, P.M. et al. C-reactive protein collaborates with plasma lectins to boost immune response against bacteria. The EMBO journal 26, 3431-3440 (2007).

Noursadeghi, M. et al. Role of serum amyloid P component in bacterial infection: protection of the host or protection of the pathogen. Proceedings of the National Academy of Sciences of the United States of America 97, 14584-14589 (2000).

Olesen, R. et al. DC-SIGN (CD209), pentraxin 3 and vitamin D receptor gene variants associate with pulmonary tuberculosis risk in West Africans. Genes and immunity 8, 456-467 (2007).

Pepys, M.B., Baltz, M., Gomer, K., Davies, A.J. & Doenhoff, M. Serum amyloid P-component is an acute-phase reactant in the mouse. Nature 278, 259-261 (1979).

Pepys, M.B. & Hirschfield, G.M. C-reactive protein: a critical update. The Journal of clinical investigation 111, 1805-1812 (2003).

Pepys, M.B. et al. Targeting C-reactive protein for the treatment of cardiovascular disease. Nature 440, 1217-1221 (2006).

Pepys, M.B. Invasive candidiasis: new insights presaging new therapeutic approaches? The Journal of infectious diseases 206, 1339-1341 (2012).

Ricklin, D. & Lambris, J.D. Complement in immune and inflammatory disorders: pathophysiological mechanisms. J Immunol 190, 3831-3838 (2013).

Rivieccio et al., Protein Expr Purif. 2007 Jan;51(1):49-58

Rosbjerg, A. et al. Complementary Roles of the Classical and Lectin Complement Pathways in the Defense against Aspergillus fumigatus. Frontiers in immunology 7, 473 (2016).

Sainz, J. et al. Dectin-1 and DC-SIGN polymorphisms associated with invasive pulmonary Aspergillosis infection. PloS one 7, e32273 (2012).

Sanjuan, M.A., Milasta, S. & Green, D.R. Toll-like receptor signaling in the lysosomal pathways. Immunological reviews 227, 203-220 (2009).

Santus et al.., Sci Immunol. 2017 Sep 22;2(15) doi: 10.1126/sciimmunol.aan2725.

Schwalbe, R.A., Dahlback, B., Coe, J.E. & Nelsestuen, G.L. Pentraxin family of proteins interact specifically with phosphorylcholine and/or phosphorylethanolamine. Biochemistry 31, 4907-4915 (1992).

Singh, P.P., Gervais, F., Skamene, E. & Mortensen, R.F. Serum amyloid P-component-induced enhancement of macrophage listericidal activity. Infection and immunity 52, 688-694 (1986).

Singh, P.P. & Kaur, S. Serum amyloid P-component in murine tuberculosis: induction kinetics and intramacrophage Mycobacterium tuberculosis growth inhibition in vitro. Microbes and infection /Institut Pasteur 8, 541-551 (2006).

Szalai, A.J. The antimicrobial activity of C-reactive protein. Microbes and infection /Institut Pasteur 4, 201-205 (2002).

Szalai, A.J., Agrawal, A., Greenhough, T.J. & Volanakis, J.E. C-reactive protein: structural biology and host defense function. Clinical chemistry and laboratory medicine : CCIM/FESCC 37, 265-270 (1999).

Takeda, K., Kaisho, T. & Akira, S. Toll-like receptors. Annual review of immunology 21, 335-376 (2003).

Tennent, G.A. et al. Transgenic human CRP is not pro-atherogenic, pro-atherothrombotic or pro-inflammatory in apoE-/- mice. Atherosclerosis 196, 248-255 (2008).

Thakur et al. , Front Microbiol. 2016 Feb 23;7:192. doi: 10.3389/fmicb.2016.00192 van den Blink, B. et al. Recombinant human pentraxin-2 therapy in patients with idiopathic pulmonary fibrosis: safety, pharmacokinetics and exploratory efficacy. Eur Respir J 47, 889-897 (2016).

Wojtowicz, A. et al. PTX3 Polymorphisms and Invasive Mold Infections After Solid Organ Transplant. Clinical infectious diseases : an official publication of the Infectious Diseases Society of America 61, 619-622 (2015).

### SEQUENCES

### SEQ ID NO:1 (APCS gene, uniprot P02743, NCBI Gene ID: 325)

| | | | | |
|---|---|---|---|---|
| 10 | 20 | 30 | 40 | 50 |
| MNKPLLWISV | LTSLLEAFAH | TDLSGKVFVF | PRESVTDHVN | LITPLEKPLQ |
| 60 | 70 | 80 | 90 | 100 |
| NFTLCFRAYS | DLSRAYSLFS | YNTQGRDNEL | LVYKERVGEY | SLYIGRHKVT |
| 110 | 120 | 130 | 140 | 150 |
| SKVIEKFPAP | VHICVSWESS | SGIAEFWING | TPLVKKGLRQ | GYFVEAQPKI |
| 160 | 170 | 180 | 190 | 200 |
| VLGQEQDSYG | GKFDRSQSFV | GEIGDLYMWD | SVLPPENILS | AYQGTPLPAN |
| 210 | 220 | | | |
| ILDWQALNYE | IRGYVIIKPL | VWV | | |

### SEQ ID NO:2 (PTX3 gene, uniprot P26022, NCBI Gene ID: 5806)

| | | | | |
|---|---|---|---|---|
| 10 | 20 | 30 | 40 | 50 |
| MHLLAILFCA | LWSAVLAENS | DDYDLMYVNL | DNEIDNGLHP | TEDPTPCDCG |
| 60 | 70 | 80 | 90 | 100 |
| QEHSEWDKLF | IMLENSQMRE | RMLLQATDDV | LRGELQRLRE | ELGRLAESLA |
| 110 | 120 | 130 | 140 | 150 |
| RPCAPGAPAE | ARLTSALDEL | LQATRDAGRR | LARMEGAEAQ | RPEEAGRALA |
| 160 | 170 | 180 | 190 | 200 |
| AVLEELRQTR | ADLHAVQGWA | ARSWLPAGCE | TAILFPMRSK | KIFGSVHPVR |
| 210 | 220 | 230 | 240 | 250 |
| PMRLESFSAC | IWVKATDVLN | KTILFSYGTK | RNPYEIQLYL | SYQSIVFWG |
| 260 | 270 | 280 | 290 | 300 |
| GEENKLVAEA | MVSLGRWTHL | CGTWNSEEGL | TSLWVNGELA | ATTVEMATGH |
| 310 | 320 | 330 | 340 | 350 |
| IVPEGGILQI | GQEKNGCCVG | GGFDETLAFS | GRLTGFNIWD | SVLSNEEIRE |
| 360 | 370 | 380 | | |
| TGGAESCHIR | GNIVGWGVTE | IQPHGGAQYV | S | |

## Claims

1. A Serum Amyloid P component (SAP) polypeptide or functional fragment of such SAP polypeptide for use in the treatment of an *Eurotiomycetes* fungus infection wherein the SAP polypeptide comprises an amino acid sequence that is at least 70% identical to SEQ ID NO: 1.

2. The SAP polypeptide or functional fragment of such SAP polypeptide for use according to claim 1, wherein the *Eurotiomycetes* fungus is an *Eurotiales* fungus.

3. The SAP polypeptide or functional fragment of such SAP polypeptide for use according to claim 2, wherein the *Eurotiales* fungus is a *Trichocomaceae* fungus.

4. The SAP polypeptide or functional fragment of such SAP polypeptide for use according to claim 2, wherein the *Eurotiales* fungus infection is selected form the list of aspergillosis and invasive aspergillosis.

5. The SAP polypeptide or functional fragment of such SAP polypeptide for use according to claims 1-4 wherein the SAP polypeptide comprises an amino acid sequence that is at least 80% identical to SEQ ID NO: 1.

6. The SAP polypeptide or functional fragment of such SAP polypeptide for use according to claims 1-4 wherein the SAP polypeptide comprises an amino acid sequence that is at least 90% identical to SEQ ID NO: 1.

7. The SAP polypeptide or functional fragment of such SAP polypeptide for use according to claims 1-4 wherein the SAP polypeptide comprises an amino acid sequence that is identical to SEQ ID NO: 1.

8. The combination of a SAP polypeptide or a functional fragment of such SAP polypeptide with a Pentraxin 3 (PTX3) polypeptide or a functional fragment of such PTX3 polypeptide for use in the treatment of a *Eurotiomycetes* fungus infection, wherein the SAP polypeptide and the PTX3 polypeptide comprise an amino acid sequence that is at least 70% identical to SEQ ID NO:1 and SEQ ID NO:2, respectively.

9. The combination for use according to claim 8 wherein the *Eurotiomycetes* fungus infection is selected from the list of aspergillosis and invasive aspergillosis.

10. The combination for use according to claims 8-9, wherein the SAP polypeptide and the PTX3 polypeptide comprise an amino acid sequence that is at least 80% identical to SEQ ID NO:1 and SEQ ID NO:2, respectively.

11. The combination for use according to claims 8-9, wherein the SAP polypeptide and the PTX3 polypeptide comprise an amino acid sequence that is at least 90% identical to SEQ ID NO:1 and SEQ ID NO:2, respectively.

12. The combination for use according to claims 8-9, wherein the SAP polypeptide and the PTX3 polypeptide comprise an amino acid sequence that is identical to SEQ ID NO: 1 and SEQ ID NO:2, respectively.

## Patentansprüche

1. Serum-Amyloid-P-Komponente(SAP)-Polypeptid oder funktionelles Fragment eines solchen SAP-Polypeptids zur Verwendung bei der Behandlung einer Infektion mit einem *Eurotiomycetes-Pilz,* wobei das SAP-Polypeptid eine Aminosäuresequenz umfasst, die zu mindestens 70 % mit SEQ ID NR. 1 identisch ist.

2. SAP-Polypeptid oder funktionelles Fragment eines solchen SAP-Polypeptids zur Verwendung nach Anspruch 1, wobei es sich bei dem *Eurotiomycetes-Pilz* um einen *Eurotiales-*Pilz handelt.

3. SAP-Polypeptid oder funktionelles Fragment eines solchen SAP-Polypeptids zur Verwendung nach Anspruch 2, wobei es sich bei dem *Eurotiomycetes-Pilz* um einen *Trichocomacea-*Pilz handelt.

4. SAP-Polypeptid oder funktionelles Fragment eines solchen SAP-Polypeptids zur Verwendung nach Anspruch 2, wobei die Infektion mit einem *Eurotiales-*Pilz aus der aus Aspergillose und invasiver Aspergillose bestehenden Liste ausgewählt ist.

5. SAP-Polypeptid oder funktionelles Fragment eines solchen SAP-Polypeptids zur Verwendung nach den Ansprüchen 1-4, wobei das SAP-Polypeptid eine Aminosäuresequenz umfasst, die zu mindestens 80 % mit SEQ ID NR. 1 identisch ist.

6. SAP-Polypeptid oder funktionelles Fragment eines solchen SAP-Polypeptids zur Verwendung nach den Ansprüchen 1-4, wobei das SAP-Polypeptid eine Aminosäuresequenz umfasst, die zu mindestens 90 % mit SEQ ID NR. 1 identisch ist.

7. SAP-Polypeptid oder funktionelles Fragment eines solchen SAP-Polypeptids zur Verwendung nach den Ansprüchen 1-4, wobei das SAP-Polypeptid eine Aminosäuresequenz umfasst, die mit SEQ ID NR. 1 identisch ist.

8. Kombination eines SAP-Polypeptids oder eines funktionellen Fragments eines solchen SAP-Polypeptids mit einem Pentraxin-3(PTX3)-Polypeptid oder einem funktionellen Fragment eines solchen PTX3-Polypeptids zur Verwendung bei der Behandlung einer Infektion mit einem *Eurotiomycetes-Pilz,* wobei das SAP-Polypeptid und das PTX3-Polypeptid eine Aminosäuresequenz umfassen, die zu mindestens 70 % mit SEQ ID NR. 1 bzw. SEQ ID Nr. 2 identisch ist.

9. Kombination zur Verwendung nach Anspruch 8, wobei die Infektion mit einem *Eurotiomycetes-Pilz* aus der aus Aspergillose und invasiver Aspergillose bestehenden Liste ausgewählt ist.

10. Kombination zur Verwendung nach den Ansprüchen 8-9, wobei das SAP-Polypeptid und das PTX3-Polypeptid eine Aminosäuresequenz umfassen, die zu mindestens 80 % mit SEQ ID NR. 1 bzw. SEQ ID NR. 2 identisch ist.

11. Kombination zur Verwendung nach den Ansprüchen 8-9, wobei das SAP-Polypeptid und das PTX3-Polypeptid eine Aminosäuresequenz umfassen, die zu mindestens 90 % mit SEQ ID NR. 1 bzw. SEQ ID NR. 2 identisch ist.

12. Kombination zur Verwendung nach den Ansprüchen 8-9, wobei das SAP-Polypeptid und das PTX3-Polypeptid eine Aminosäuresequenz umfassen, die mit SEQ ID NR. 1 bzw. SEQ ID NR. 2 identisch ist.

## Revendications

1. Polypeptide SAP (composant amyloïde P sérique) ou fragment fonctionnel d'un tel polypeptide SAP pour son utilisation dans le traitement d'une infection par un champignon Eurotiomycetes dans lequel le polypeptide SAP comprend une séquence d'acides aminés qui est identique à 70 % avec SEQ ID NO : 1.

2. Polypeptide SAP ou fragment fonctionnel d'un tel polypeptide SAP pour son utilisation selon la revendication 1, dans lequel le champignon Eurotiomycetes est un champignon Eurotiales.

3. Polypeptide SAP ou fragment fonctionnel d'un tel polypeptide SAP pour son utilisation selon la revendication 2, dans lequel le champignon Eurotiales est un champignon Trichocomaceae.

4. Polypeptide SAP ou fragment fonctionnel d'un tel polypeptide SAP pour son utilisation selon la revendication 2, dans lequel l'infection par le champignon Eurotiales est sélectionnée dans la liste de l'aspergillose et l'aspergillose invasive.

5. Polypeptide SAP ou fragment fonctionnel d'un tel polypeptide SAP pour son utilisation selon les revendications 1 à 4 dans lequel le polypeptide SAP comprend une séquence d'acides aminés qui présente une identité d'au moins 80 % avec SEQ ID NO : 1.

6. Polypeptide SAP ou fragment fonctionnel d'un tel polypeptide SAP pour son utilisation selon les revendications 1 à 4 dans lequel le polypeptide SAP comprend une séquence d'acides aminés qui présente une identité d'au moins 90 % avec SEQ ID NO : 1.

7. Polypeptide SAP ou fragment fonctionnel d'un tel polypeptide SAP pour son utilisation selon les revendications 1 à 4 dans lequel le polypeptide comprend une séquence d'acides aminés qui est identique à SEQ ID NO : 1.

8. Combinaison d'un polypeptide SAP ou d'un fragment fonctionnel de ce polypeptide SAP avec un polypeptide PTX3 (Pentraxine 3) ou un fragment fonctionnel d'un tel polypeptide PTX3 pour son utilisation dans le traitement d'une infection par le champignon Eurotiomycetes, dans laquelle le polypeptide SAP et le polypeptide PTX3 comprennent une séquence d'acides aminés qui présente une identité d'au moins 70 % avec SEQ ID NO : 1 et SEQ ID NO : 2, respectivement.

9. Combinaison pour son utilisation selon la revendication 8 dans laquelle l'infection par le champignon Eurotiomycetes est sélectionnée parmi l'aspergillose et l'aspergillose invasive.

10. Combinaison pour son utilisation selon les revendications 8 et 9, dans laquelle le polypeptide SAP et le polypeptide PTX3 comprennent une séquence d'acides aminés qui présente une identité d'au moins 80 % avec SEQ ID NO : 1 et SEQ ID NO : 2, respectivement.

11. Combinaison pour son utilisation selon les revendications 8 et 9, dans laquelle le polypeptide SAP et le polypeptide PTX3 comprennent une séquence d'acides aminés qui présente une identité d'au moins 90 % avec SEQ ID NO : 1 et SEQ ID NO : 2, respectivement.

12. Combinaison pour son utilisation selon les revendications 8 et 9, dans laquelle le polypeptide SAP et le polypeptide PTX3 comprennent une séquence d'acides aminés qui est identique à SEQ ID NO : 1 et SEQ ID NO : 2, respectivement.
